(19) Europäisches Patentamt
European Patent Office
Office européen des brevets

(11) EP 1 826 278 A1

(12) EUROPEAN PATENT APPLICATION

(43) Date of publication:
**29.08.2007 Bulletin 2007/35**

(51) Int Cl.:
***C12Q 1/68*** *(2006.01)*

(21) Application number: **07004137.1**

(22) Date of filing: **28.02.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **28.02.2006 US 777631 P**

(71) Applicant: **Epiontis GmbH**
**12489 Berlin (DE)**

(72) Inventors:
- **Olek, Sven**
  **12207 Berlin (DE)**
- **Türbachova, Ivana**
  **12207 Berlin (DE)**

(74) Representative: **Krauss, Jan**
**Forrester & Boehmert,**
**Pettenkoferstrasse 20-22**
**80336 München (DE)**

# (54) Epigenetic modification of the loci for camta1 and/or foxp3 as a marker for cancer treatment

(57) The present invention relates to a method, in particular an *in vitro* method, for pan-cancer diagnostics, comprising identifying the amount and/or proportion of stable regulatory T cells in a patient suspected of having cancer through analyzing the methylation status of at least one CpG position in the gene foxp3 and/or the gene camtal or orthologous or paralogous genes thereof, wherein an increased amount and/or proportion of stable regulatory T cells in said patient is indicative for an unspecific cancerous disease. In a second aspect thereof, the present invention relates to a method for diagnosing the survival of a cancer patient, comprising identifying the amount and/or proportion of stable regulatory T cells in said cancer patient through analyzing the methylation status of at least one CpG position in the gene foxp3 and/or the gene camtal or orthologous or paralogous genes thereof, wherein a demethylation in the gene foxp3 and/or the gene camtal or orthologous or paralogous genes thereof, is indicative of a stable regulatory T cell, and wherein an increased amount and/or proportion of stable regulatory T cells in said cancer patient is indicative for a shorter survival for said cancer patient. Furthermore, the present invention relates to an improved treatment of cancers based on the inventive methods, and a kit for performing the above methods as well as respective uses.

EP 1 826 278 A1

## Description

The present invention relates to a method, in particular an *in vitro* method, for pan-cancer diagnostics, comprising identifying the amount and/or proportion of stable regulatory T cells in a patient suspected of having cancer through analyzing the methylation status of at least one CpG position in the gene foxp3 and/or the gene camta1 or orthologous or paralogous genes thereof, wherein an increased amount and/or proportion of stable regulatory T cells in said patient is indicative for an unspecific cancerous disease. In a second aspect thereof, the present invention relates to a method for diagnosing the survival of a cancer patient, comprising identifying the amount and/or proportion of stable regulatory T cells in said cancer patient through analyzing the methylation status of at least one CpG position in the gene foxp3 and/or the gene camta1 or orthologous or paralogous genes thereof, wherein a demethylation in the gene foxp3 and/or the gene camta1 or orthologous or paralogous genes thereof, is indicative of a stable regulatory T cell, and wherein an increased amount and/or proportion of stable regulatory T cells in said cancer patient is indicative for a shorter survival for said cancer patient. Furthermore, the present invention relates to an improved treatment of cancers based on the inventive methods, and a kit for performing the above methods as well as respective uses.

For the purpose of the present invention all references as cited herein as well as the sequence listing are incorporated by reference in their entireties.

## Background of the invention

Regulatory T cells play an important role for the maintenance of immunological tolerance by suppressing the action of autoreactive effector cells, making them interesting targets for therapeutic applications. Therefore, these cells are critically involved in preventing the development of autoimmune reactions (Sakaguchi, Nat Immunol 6:345-352, 2005). Regulatory T cells (Tregs), which have been shown to play a pivotal role in the maintenance of self-tolerance within the immune system, were described originally as CD4+ T cells constitutively expressing CD25 (Sakaguchi, S. Naturally arising CD4+ regulatory T cells for immunologic selftolerance and negative control of immune responses. Annu Rev Immunol 22, 531-562 (2004)). More recently, the forkhead transcription factor Foxp3 has been shown to be specifically expressed in Tregs and to be a central control element in Treg development and function (Fontenot, J.D. and Rudensky, A.Y. A well adapted regulatory contrivance: regulatory T cell development and the forkhead family transcription factor Foxp3. Nat Immunol 6, 331-7 (2005)). Mutation or deletion of the gene encoding Foxp3 causes severe autoimmune disease in mice and humans, due to a failure to generate CD25+CD4+ Tregs (Bennett, C.L. et al. The immune dysregulation, polyendocrinopathy, enteropathy, X-linked syndrome (IPEX) is caused by mutations of FOXP3. Nat Genet 27, 20-1 (2001). Fontenot, J.D., Gavin, M.A. and Rudensky, A.Y. Foxp3 programs the development and function of CD4+CD25+ regulatory T cells. Nat Immunol 4, 330-6 (2003)), whereas ectopic expression of Foxp3 in conventional T cells confers suppressive activity (Fontenot, J.D., Gavin, M.A. and Rudensky, A.Y. Foxp3 programs the development and function of CD4+CD25+ regulatory T cells. Nat Immunol 4, 330-6 (2003). Hori, S., Nomura, T. and Sakaguchi, S. Control of regulatory T cell development by the transcription factor Foxp3. Science 299, 1057-61 (2003)).

These findings provided compelling evidence that Foxp3 acts as a master switch controlling the development and function of Tregs; however, the molecular mechanisms leading to its induction remain largely unknown. Recently, an initial characterization of the human FOXP3 promoter revealed a basal, T cell-specific promoter containing several NF-AT and AP-1 binding sites, which are positively regulating FOXP3 expression after triggering of the TCR (Mantel, P.Y. et al. Molecular Mechanisms Underlying FOXP3 Induction in Human T Cells. J Immunol 176, 3593-602 (2006)).

Occurrence of autoimmunity in thymectomized mice provided initial evidence that Foxp3+CD25+CD4+ Tregs are generated as an individual lineage within the thymus 1. Using mice harbouring a GFP-Foxp3 fusion protein-reporter knockin allele, Fontenot et al. could show that Foxp3 expression becomes prominent in CD4 single positive (SP) thymocytes (~83% of $Foxp3^{gfp+}$ thymocytes) ( Fontenot, J.D. et al. Regulatory T cell lineage specification by the forkhead transcription factor foxp3. Immunity 22, 329-41 (2005). Fontenot, J.D., Dooley, J.L., Farr, A.G. and Rudensky, A.Y. Developmental regulation of Foxp3 expression during ontogeny. J Exp Med 202, 901-6 (2005)). In addition to the thymic generation of Foxp3+ Tregs, peripheral conversion of Foxp3-CD25-CD4+ T cells into Foxp3+ Tregs has recently been demonstrated by tolerogenic antigen application in vivo (Thorstenson, K.M. and Khoruts, A. Generation of anergic and potentially immunoregulatory CD25+CD4 T cells in vivo after induction of peripheral tolerance with intravenous or oral antigen. J Immunol 167, 188-95. (2001). Apostolou, I. and von Boehmer, H. In vivo instruction of suppressor commitment in naive T cells. J Exp Med 199, 1401-8 (2004). Kretschmer, K. et al. Inducing and expanding regulatory T cell populations by foreign antigen. Nat Immunol 12, 1219-27 (2005)) or upon activation in the presence of TGF-$\beta$ in vitro (Chen, W. et al. Conversion of peripheral CD4+CD25- naive T cells to CD4+CD25+ regulatory T cells by TGF-beta induction of transcription factor Foxp3. J Exp Med 198, 1875-86 (2003). Park, H.B., Paik, D.J., Jang, E., Hong, S. and Youn, J. Acquisition of anergic and suppressive activities in transforming growth factor-beta-costimulated CD4+CD25-T cells. Int Immunol 16, 1203-13 (2004). Fu, S. et al. TGF-beta induces Foxp3 + T-regulatory cells from CD4 + CD25 -precursors. Am J Transplant 4, 1614-27 (2004). Fantini, M.C. et al. Cutting edge: TGF-beta induces a regulatory phenotype in CD4+CD25- T cells through Foxp3 induction and down-regulation of Smad7. J Immunol 172, 5149-53 (2004). Wan, Y.Y. and Flavell, R.A. Identifying Foxp3-expressing suppressor T cells with a bicistronic reporter. Proc Natl Acad Sci U S A 102, 5126-31 (2005). Fantini, M.C. et al. TGF-{beta} induced Foxp3+ regulatory T cells suppress Th1-mediated experimental colitis. Gut (2005)). To what extent these induced populations of Tregs acquire a stable phenotype corresponding to that of natural, thymus-derived Tregs is, however, unclear.

An emerging paradigm in understanding the development of stable cellular lineages emphasizes the role of epigenetic mechanisms for the permanent, heritable fixation of distinct gene expression patterns. Molecular mechanisms of epigenetic imprinting include selective demethylation of CpG motifs and histone modifications as shown for cytokine genes (Ansel, K.M., Lee, D.U. and Rao, A. An epigenetic view of helper T cell differentiation. Nat Immunol 4, 616-23 (2003). Reiner, S.L. Epigenetic control in the immune response. Hum Mol Genet 14 Spec No 1, R41-6 (2005). Tykocinski, L.O. et al. A critical control element for interleukin-4 memory expression in T helper lymphocytes. J Biol Chem 280, 28177-85 (2005)).

WO 02/090600 describes isolated nucleic acid molecules which encode Fkhsf, as well as mutant forms thereof. Also described are expression vectors suitable for expressing such nucleic acid molecules, and host cells containing such expression vectors. Also described are pharmaceutical compounds and methods of identifying such compounds that can modulate the immune system. In addition are provided methods for identifying proteins regulated by Scurfin and proteins that induce or inhibit Foxp3 expression.

Chen et al. (Chen L, Cohen AC, Lewis DB. Impaired Allogeneic Activation and T-helper 1 Differentiation of Human Cord Blood Naive CD4 T Cells. Biol Blood Marrow Transplant. 2006 Feb;12(2):160-71.) describe FoxP3 protein expression as a marker for regulatory CD25(high) CD4 T cells.

Bouche et al. (in Bouche, N., Scharlat, A., Snedden, W., Bouchez, D. and Fromm, H. A novel family of calmodulin-binding transcription activators in multicellular organisms J. Biol. Chem. 277 (24), 21851-21861 (2002)) describe two human proteins designated HsCAMTA1 and HsCAMTA2 as members of a conserved family of transcription factors in a wide range of multicellular eukaryotes, which possibly respond to calcium signaling by direct binding of calmodulin.

Henrich et al. (in K. O. Henrich, M. Fischer, D. Mertens, A. Benner, R. Wiedemeyer, B. Brors, A. Oberthuer, F. Berthold, J. S. Wei, J. Khan, M. Schwab, and F. Westermann. Reduced expression of CAMTA1 correlates with adverse outcome in neuroblastoma patients. Clin Cancer Res, 12(1):131-138, January 2006.) describe that the reduced expression of CAMTA1 correlates with adverse outcome in neuroblastoma patients.

Even though almost all cells in an individual contain the exact same complement of DNA code, higher organisms must impose and maintain different patterns of gene expression in the various tissue types. Most gene regulation is transitory, depending on the current state of the cell and changes in external stimuli. Persistent regulation, on the other hand, is a primary role of epigenetics - heritable regulatory patterns that do not alter the basic genetic coding of the DNA. DNA methylation is the archetypical form of epigenetic regulation; it serves as the stable memory for cells and performs a crucial role in maintaining the long-term identity of various cell types.

The primary target of methylation is the two-nucleotide sequence Cytosine-Guanine (a 'CpG site'); within this context cytosine (C) can undergo a simple chemical modification to become 5-methyl-cytosine. In the human genome, the CG sequence is much rarer than expected except in certain relatively dense clusters called 'CpG islands'. CpG islands are frequently associated with gene promoters, and it has been estimated that more than half of the human genes have CpG islands (Antequera and Bird, Proc Natl Acad Sci U S A. 90:11995-9, 1993).

Aberrant methylation of DNA frequently accompanies the transformation from healthy to cancerous cells. Among the observed effects are genome-wide hypomethylation, increased methylation of tumour suppressor genes and hypomethylation of many oncogenes (reviewed by Jones and Laird, Nature Genetics 21:163-167, 1999; Esteller, Oncogene 21:5427-5440, 2002; Laird, Nature Reviews/Cancer 3:253-266, 2003). Methylation profiles have been recognised to be tumour specific (i.e., changes in the methylation pattern of particular genes or even individual CpGs are diagnostic of particular tumour types) and there is now an extensive collection of diagnostic markers for bladder, breast, colon, oesophagus, stomach, liver, lung, and prostate cancers (summarised by Laird, Nature Reviews/Cancer 3:253-266, 2003).

Epigenetic control by methylation is essential for early development including embryogenesis, X-chromosome inactivation and imprinting (monoallelic silencing) of either the paternal or maternal allele (Erlich, J Cellular Chem 88: 899-910, 2003). There is also a class of genes that is active in the germ line, but is silenced by methylation in somatic cells (Bird, Genes and Dev 16:6-21, 2002; Li, Nature Reviews/Genetics 3:662-673, 2002).

Tissue-specific methylation also serves in regulating adult cell types/stages, and in some cases a causal relationship between methylation and gene expression has been established. The following is a partial list of genes, for which methylation changes are strongly implicated in controlling gene expression in tissue-specific manner: Lactate dehydrogenase C (testes); Oxytocin receptor (blood and liver); Tyrosine aminotransferase (liver); GFAP (astrocytes); and Leukosialin (leukocytes). In other cases, methylation may be a by-product of some other primary regulation, or it is required to lock the gene in the "off" state (Erlich, J Cellular Chem 88:899-910, 2003). For the present applications (cell/tissue identification), a causal relationship is not required, merely a strong correlation between methylation patterns and cell types.

A previously published example for such a cell type and cell status specific modification of certain gene regions is found during the lineage commitment of T cells to helper T cells (Th1 or Th2). Naive (unstimulated) $CD4^+$ T cells become activated upon encountering an antigen and can be committed to alternative cell fates through further stimulation by interleukins. The two types of helper T cells show reciprocal patterns of gene expression; Th1 produces Interferon-gamma (IFN-$\gamma$) and silences IL-4, while Th2 produces IL-4 and silences IFN-$\gamma$ (Ansel et al., Nature Immunol 4:616-623, 2003). For both alternative cell fates, the expression of these genes is inversely correlated with methylation of proximal CpG sites. In Th2 and naive T cells the IFN- $\gamma$ promoter is methylated, but not in Th1 cells where IFN-$\gamma$ is expressed (Attwood et al., CMLS 59:241-257, 2002). Conversely, the entire transcribed region of IL-4 becomes demethylated under Th2-inducing conditions, which strongly correlates with efficient transcription of IL-4. In Th1 cells, this extensive demethylation does not occur, rather particular untranscribed regions gradually become heavily methylated and IL-4 is not expressed (Lee et al., Immunity 16:649-660, 2002). Furthermore, Bruniquel and Schwartz (Nat Immunol. 4:235-40, 2003) have demonstrated that in naive T cells, the IL-2 promoter is heavily methylated and inactive, but after activation of the naive T cell, the IL-2 gene undergoes rapid and specific demethylation at six consecutive CpGs. This alteration in methylation patterns occurs concomitantly with cell differentiation and increased production of the IL-2 product.

Dietl et al. (in Dietl J, Engel JB, Wischhusen J. The role of regulatory T cells in ovarian cancer. Int J Gynecol Cancer. 2007 Feb 14) describe that an increased mortality rate is associated with the presence of a high number of intratumoral regulatory T-cells. Tumor infiltration by non-regulatory T-cells, on the other hand, is predictive for a substantially longer patient survival. The article further reviews clinical and experimental findings on Tregs in ovarian cancer, with special regard to potential therapeutic implications, which may result from the existing evidence.

Curiel et al. (in Curiel TJ, Coukos G, Zou L, Alvarez X, Cheng P, Mottram P, Evdemon-Hogan M, Conejo-Garcia JR, Zhang L, Burow M, Zhu Y, Wei S, Kryczek I, Daniel B, Gordon A, Myers L, Lackner A, Disis ML, Knutson KL, Chen L, Zou W. Specific recruitment of regulatory T cells in ovarian carcinoma fosters immune privilege and predicts reduced survival. Nat Med. 2004 Sep;10(9):942-9. Epub 2004 Aug 22) show in studies of CD4(+)CD25(+)FOXP3(+) T (reg) cells in 104 individuals affected with ovarian carcinoma, that human tumor T(reg) cells suppress tumor-specific T cell immunity and contribute to growth of human tumors in vivo. Blocking T(reg) cell migration or function is proposed in order to help to defeat human cancer.

Kobayashi et al. (in Kobayashi N, Hiraoka N, Yamagami W, Ojima H, Kanai Y, Kosuge T, Nakajima A, Hirohashi S. FOXP3+ regulatory T cells affect the development and progression of hepatocarcinogenesis. Clin Cancer Res. 2007 Feb 1;13(3):902-11) describe that CD4+CD25+FOXP3+ regulatory T cells (Tregs) suppress the immune reaction, and the clinicopathologic significance and roles of Tregs and CD8+ T cells during hepatocarcinogenesis was examined. The patient group with a high prevalence of Tregs infiltrating HCC showed a significantly lower survival rate (P=0.007). Multivariate analysis revealed that the prevalence of Tregs infiltrating HCC was an independent prognostic factor. A high prevalence of Tregs infiltrating HCC is described to be an unfavorable prognostic indicator.

It is an object of the present invention, to provide an improved method of cancer diagnostics based on the DNA methylation analysis and, optionally, packaging of the chromatin structure as a superior tool that can supplement or replace conventional methodologies, in order to reliably identify FoxP3-positive regulatory T cells, preferably $CD25^+CD4^+$ regulatory T cells, of a mammal and/or in a mammal. Based on the presence or absence of said FoxP3-positive regulatory T cells, preferably $CD25^+CD4^+$ regulatory T cells, improved options for the treatment of cancerous diseases can be provided and employed.

## Summary of the invention

According to a first aspect thereof, the present invention solves the above object by providing a method for pan-cancer diagnostics, comprising identifying the amount and/or proportion of stable regulatory T cells in a patient suspected of having cancer through analysing the methylation status of at least one CpG position in the gene foxp3 and/or the gene camta1 or orthologous or paralogous genes thereof, wherein an increased amount and/or proportion of stable regulatory T cells in said patient is indicative for an unspecific cancerous disease.

As used herein, the term "patient suspected of having cancer" refers to a subject that presents one or more symptoms indicative of a cancer (e.g., a noticeable lump or mass). A patient suspected of having cancer may also have on or more risk factors. A patient suspected of having cancer has generally not been tested for cancer. However, a "patient suspected of having cancer" encompasses an individual who has received an initial diagnosis (e.g., a CT scan showing a mass) but for whom the sub-type or stage of cancer is not known. The term further includes patients who once had cancer (e.g., an individual in remission).

The analysis of foxp3 and/or camta1 as a so-called "pan-cancer markers" has the advantage that they can be very sensitive and specific for a kind of "cancer-yes/no" information, but at the same time need not to give a clear indication about the localization of the cancer (e.g. need not to be tissue- and/or cell-specific). Thus, this allows for a simplified generation of qualitative and improved diagnostic marker panels for proliferative diseases, since a sensitive and, for example, a very tissue-specific marker can be combined in such a diagnostic marker panel. Nevertheless, the present method according to the invention, in particular in embodiments for following-up (monitoring) of once identified proliferative diseases, can also include a quantitative analysis of the expression and/or the methylation of the markers as employed. A preferred embodiment of the method according to the invention thus further comprises the analysis of at least one cancer- and/or tissue-specific marker. Respective markers are well known in the literature. Thus, for a localization of the cancer/determination of the type of cancer, a detection of specific tissue markers based on, e.g., nucleic acid-analysis is performed, and the two results of the marker analyses are combined in order to provide a localization of the cancer/determination of the type of cancer (characterization thereof).

The term "pan-cancer marker" refers to a marker (in the present case, foxP3 and/or camta1) that may be detectable if present in blood, serum or other bodily fluids, or preferably in tissues that is reflective of the presence of proliferative disease. Pan-cancer markers are characterized by the fact that they reflect the possibility of the presence of more than one, preferably a multitude of, proliferative diseases in organs or tissues of the patient and/or subject. Thus, pan-cancer markers are not specific for a single proliferative disease being present in an organ or tissue, but are specific for more than one proliferative disease for said patient. The term may alternately refer to a specific characteristic of said marker, such as, but not limited to, a specific methylation pattern, making the marker distinguishable from otherwise identical markers. Typically, this marker is derived from the tumor itself.

According to a another important aspect thereof, the present invention solves the above object by providing a method for diagnosing the survival of a cancer patient, comprising identifying the amount and/or proportion of stable regulatory T cells in said cancer patient through analysing the methylation status of at least one CpG position in the gene foxp3 or an orthologous or paralogous gene thereof, wherein a demethylation in the gene foxp3 and/or camta1 or orthologous or paralogous genes thereof, is indicative of a stable regulatory T cell, and wherein an increased amount and/or proportion of stable regulatory T cells in said cancer patient is indicative for a shorter survival for said cancer patient.

Preferably, said stable regulatory T cell is a $CD25^+CD4^+$ regulatory T cell.

In the context of the present invention, it was surprisingly found that the methylation status of at least one CpG position in the gene camta1 is indicative of a stable regulatory T cell. Thus, according to a second aspect thereof, the present invention solves the above object by providing a method for identifying Camtal-positive regulatory T cells, preferably $CD25^+CD4^+$ regulatory T cells of a mammal, comprising analysing the methylation status of at least one CpG position in the gene camta1 or an orthologous or paralogous gene thereof. Said method can be performed *in vitro* and/or *in vivo.* Preferred is a method according to the invention, wherein said Camta1-positive regulatory T cells, preferably $CD25^+CD4^+$ regulatory T cells are stable Camta1-positive regulatory T cells, preferably stable $CD25^+CD4^+$ regulatory T cells. The analysis of the accessibility of the Camta1 locus provides additional information, besides the mere expression of Camta1, to what extent a permanent conversion into a regulatory T cell lineage has occurred.

More preferably, said analysis of the methylation status according to the invention comprises analysing the methylation status of at least one CpG position in the 5' region upstream from the transcription start, promoter regions, introns, and/or exon/intron borders of the gene foxp3 and/or camta1.

The inventors have identified particular regions within the Foxp3 and the Camta1 gene, which are functionally involved in the regulation of stable Foxp3 and Camta1 expression, respectively, in regulatory T cells. These regions contain many CpG motifs, which display a differential methylation status when cells expressing Camta1 and/or Foxp3 (preferably $CD25^+CD4^+$) compared with cells not expressing Camta1 and/or Foxp3 (preferably $CD25^-CD4^+$), if, for example, the bisulphite sequencing method is used. The inventors could demonstrate that in $Foxp3^+$ and/or $Camta1^+$ cells the CpG motifs are almost completely demethylated (i.e. to more than 70%, preferably 80%, preferably, more than 90% and most preferred more than 95%), whereas the same motifs are completely methylated in $Foxp3^-$ and/or Camta $1^-$ cells. The differential methylation of the CpG motifs within the aforementioned region strongly correlates with Foxp3 and/or Camta1 expression. Thus, determination of the methylation status of the *foxp3* and/or *camta1* loci could become a valuable tool to identify stable populations of regulatory T cells required for clinical application in the treatment of cancer, autoimmune disease, transplant rejection or allergy.

Further preferred is an analysis of the methylation status according to the invention that comprises analysing the methylation status of at least one CpG position in the 5' region upstream from the transcription start, promoter regions, introns, and/or exon/intron borders of the gene foxp3 and/or camta1.

Whether Treg differentiation also involves elements of epigenetic regulation has not been studied thus far. The inventors therefore investigated whether epigenetic alterations such as DNA methylation and histone modifications of the foxp3 and/or camta1 loci correlate with Foxp3 and Camta1 expression, respectively. The selective association of chromatin remodelling with a stable Treg phenotype established a role of epigenetic imprinting in the establishment of a committed regulatory cell type.

The inventors have identified particular regions within the Foxp3 and Camta1 gene, which are functionally involved in the regulation of stable Foxp3 or Camta1 expression in regulatory T cells. This region contains many CpG motifs, which display a differential methylation status when cells expressing Foxp3 and/or Camta1 (preferably $CD25^+CD4^+$) compared with cells not expressing Foxp3 and/or Camta1 (preferably $CD25^-CD4^+$), if, for example, the bisulphite sequencing method is used. The inventors could demonstrate that in $Foxp3^+$ and $Camta1^+$ cells the CpG motifs are almost completely demethylated (i.e. to more than 70%, preferably 80%, preferably, more than 90% and most preferred more than 95%), whereas the same motifs are completely methylated in Foxp3$^-$ and/or Camta1$^-$ cells. The differential methylation of the CpG motifs within the aforementioned region strongly correlates with Foxp3 and/or Camta1 expression. Thus, determination of the methylation status of the *foxp3* and/or the *camta1* locus could become a valuable tool to identify the amount and/or proportion of stable regulatory T cells in order to diagnose the survival of a cancer patient.

Preferred is a combined analysis of both markers according to the present invention.

In the context of the present invention, the term "gene" shall mean a region of the chromosomal DNA that encodes for a certain protein, such as FoxP3 or Camta1, and contains other genetic elements that are responsible for the regulation of said gene. Thus, a gene includes also introns, enhancers, promoter sequences and the 5' untranslated region of the gene. In the present case, the gene will not only include the sequence as given in Brunkow et al. (Brunkow ME, Jeffery EW, Hjerrild KA, Paeper B, Clark LB, Yasayko SA, Wilkinson JE, Galas D, Ziegler SF, Ramsdell F. Disruption of a new forkhead/winged-helix protein, scurfin, results in the fatal lymphoproliferative disorder of the scurfy mouse. Nat Genet. 2001 Jan;27(1):68-73. Accession number AF277993) or Bouche et al. (Bouche, N., Scharlat, A., Snedden, W., Bouchez, D. and Fromm, H. A novel family of calmodulin-binding transcription activators in multicellular organisms J. Biol. Chem. 277 (24), 21851-21861 (2002), Accession number NP_056030), but also includes the untranslated regions upstream and downstream thereof, as depicted in Figures 2 and 7, and as available in the database under Accession number NC_000023.

In the context of the present invention, this fact is described by the terms "orthologous" or "paralogous" gene. An "ortholog" is a gene in two or more species that has evolved from a common ancestor, and is also called an orthologous gene. In the context of the present invention, *Homo sapiens* FoxP3 (forkhead box P3) is therefore an ortholog of the *Mus musculus* Foxp3 (forkhead box P3) gene and/or protein. Other orthologs are *C. familiaris* LOC491876 (similar to Forkhead box protein P3), and *R. norvegicus* LOC317382 (similar to scurfin). "Paralogs" are genes related by duplication within a genome and are also called a paralogous gene. Orthologs retain the same function in the course of evolution, whereas paralogs evolve new functions, even if these are related to the original one. Included in the term "paralog" is a "pseudogene" that is a nucleotide sequences that is similar to a normal gene, but does not produce a functional final product. There are two variants of pseudogenes. The first requires the final product to be a protein. The second allows the final product to be an RNA.

Based on the information as given herein, the person of skill will be readily able, to compare the orthologous or paralogous genes (for example using a computer program in order to align the sequences, such as the fasta program), and to identify regions and/or CpG positions that can be found in the same regions and/or even at the same equivalent positions in the (both) genes. According to the present invention, these regions and/or CpG positions are regarded as orthologous or paralogous. Usually, an alignment is based on the level of sequence identity between the two (or more) DNA fragments that are analyzed. As a preferred example for foxp3 according to the present invention, a 384-bp stretch that is differentially methylated in the region as covered by amplicons 1 and 2 (as described herein), but not the region covered by amplicons 3 and 4 is highly conserved between mice and men (80.7% sequence identity), suggesting that in both mammals functionally important parts can be found that are both subject to epigenetic regulation. Other levels of sequence identity for the markers are preferably about 75%, more preferably about 80%, and most preferred about 90% of a given fragment.

In a preferred embodiment of the method according to the present invention, said analysis of the methylation status for foxp3 comprises amplification with at least one of the primer pairs selected from SEQ ID No. 1 and 2; SEQ ID No. 3 and 4, and orthologous or paralogous primer pairs thereof.

Preferably, the amplification involves a polymerase enzyme, a PCR or chemical amplification reaction, or other amplification methods as known to the person of skill as described below, e.g. in the context of MSP, HeavyMethyl, or MethyLight. With the amplification, amplicons 1 and 2 (as described herein exemplary for foxp3) or orthologs or paralogs thereof are produced that are particularly preferred "tools" for performing the method(s) according to the present invention.

Consequently, an oligomer according to any of SEQ ID No. 1 to 4 or an amplicon as amplified by a primer pair selected from SEQ ID No. 1 and 2, SEQ ID No. 3 and 4, or orthologous or paralogous oligomers or amplicons constitute preferred embodiments of the present invention. The same applies for camta1.

Based on the above information and the data as obtained from the murine and/or human system, orthologous or paralogous primer pairs can be designed by the person of skill having a sequence identity with the above primers of preferably about 75%, more preferably about 80%, and most preferred about 90%.

Even more preferred is a method according to the present invention, wherein said analysis of the methylation status for foxp3 comprises analysing the methylation status of at least one CpG position selected from the group consisting of positions 38, 74, 90, 124, 151, 156, 205, 224, 228, 236, 298, and 368 of the amplicon as amplified by the primer pair SEQ ID No. 1 and 2, positions 158, 180, 308, 344, 360, 394, 421, and 426 of the amplicon as amplified by the primer pair SEQ ID No. 3 and 4, and orthologous or paralogous CpG positions thereof (as also can be taken from Figure 2).

The person of skill will furthermore be able to select specific subsets of CpG positions in both genes in order to minimise the amount of sites to be analyzed, for example all sites as present on amplicon 1 or all sites as present on amplicon 2 or orthologous or paralogous CpG positions thereof.

In order to analyze the methylation status of CpG positions, any known method to analyse DNA methylation can be used. In a preferred embodiment of the method according to the present invention, the analysis of the methylation status comprises a method selected from methylation specific enzymatic digests, bisulphite sequencing, analysis selected from promoter methylation, CpG island methylation, MSP, HeavyMethyl, MethyLight, Ms-SNuPE or other methods relying on a detection of amplified DNA. These methods are well known to the person of skill, and can be found in the respective literature.

Furthermore, preferred is a method according to the invention, further comprising the step of analysing the packaging of the chromatin structure in the region of the gene foxp3 or an orthologous or paralogous gene thereof, wherein an open chromatin structure in the region of the gene foxp3 and/or camta1 or orthologous or paralogous genes thereof, is indicative of a stable regulatory T cell. In order to analyze the packaging of the chromatin structure, any known method to analyse packaging of the chromatin structure can be used. In a preferred embodiment of the method according to the present invention, the analysis of the packaging of the chromatin structure comprises chromatin immunoprecipitation (ChIP), in particular using antibodies against acetylated histones, in particular H3 and/or H4, as described herein below.

The method according to the present invention can be performed with any mammal having the foxp3 and/or camta1 gene or orthologs or paralogs thereof, preferred is a method according to the present invention, wherein said mammal is a mouse, rat, monkey or human, preferably a human.

The method according to the present invention can be performed with any mammal having the foxp3 and/or camta1 gene or orthologs or paralogs thereof, preferred is a method according to the present invention, wherein said cancer is selected from solid tumors, breast cancer, ovarian cancer, prostate cancer, and lung cancer.

Further preferred is a method, wherein the amount of regulatory T-cells corresponds to a demethylation of the CpG positions as analyzed to at least 80%, preferably 90%, and more preferably 95%.

Further preferred is a method, wherein the amount of regulatory T-cells corresponds to a demethylation as above, and an open chromatin structure in the region of the gene foxp3 and/or camta1 or orthologous or paralogous genes thereof corresponds to at least 10-fold of DNA relative to input for $Me_3K4$.

The method(s) according to the present invention can be performed *in vitro* and/or *in vivo.* In general, all biological samples can be used, as long as they contain suitable T-cells. Preferred is a method, wherein said sample is selected from a tissue sample and/or a tumour sample (containing, e.g., infiltrating T-cells), a blood sample, a sample of blood lymphocytes or a fraction thereof.

Further preferred is a method, wherein the amount and/or proportion of stable regulatory T cells is identified through a comparison with the methylation status of at least one CpG position in the gene foxp3 and/or camta1 or orthologous or paralogous gense thereof in samples selected from all cells as present in said sample, all T-cells as present in said sample, and the number of stable regulatory T cells in a healthy patient.

In one aspect of this particular method, the total population of T cells in a sample (containing regulatory and non-regulatory T-cells) is analyzed for their methylation status and, optionally, the chromatin packaging in the foxp3 and/or camta1 gene. Based on the result of the overall methylation frequency of the sites and the chromatin packaging, the ratio and/or amount of regulatory T cells inside the analyzed population can be determined. From said result, it can be concluded on the immune status and/or T cell status of the mammal as diagnosed. Preferred is a method, wherein an amount of regulatory T-cells corresponds to a demethylation of the CpG positions as analyzed to at least 80%, preferably 90%, and more preferably 95%.

Another important aspect of the present invention then relates to a method according to the present invention, which further comprises the step of providing a treatment for said cancer patient wherein said treatment reduces the amount and/or proportion of stable regulatory T cells in said cancer patient. Preferred is a method according to the present invention, wherein said treatment is selected from providing chemical and/or biological substances that selectively kill regulatory T-cells in said patient, or a treatment that reduces the expression of the gene foxp3 or inhibits the biological activity of FoxP3 and/or Camta1 in said regulatory T-cells in said patient. Preferred examples of such treatments are selected from an antibody that is selective for regulatory T-cells, such as an anti-CD25-antibody, a cytotoxic substance that is selective for regulatory T-cells, such as denileukin diftitox (Ontak®), antisense nucleic acids against the expression of the gene foxp3 and/or camta1, and methylating agents that provide for an increased methylation of the gene foxp3 and/or camta1.

Even further preferred is a method according to the invention which further comprises measuring and/or monitoring the amount or ratio of said regulatory T cells in response to the chemical and/or biological substances as mentioned above. That is, changes in the amount or ratio of regulatory T cells that are caused by, for example, the treatment of a disease (e.g. as described herein), and the success and/or progress of said treatment in terms of an effect on regulatory T cells can be followed using this method. A follow-up of the methylation pattern and, optionally, the chromatin structure of T cells based on the marker herein will point to changes in the cells that are due to a response to said chemical and/or biological substances, in some cases even before a phenotypic change can be observed.

In yet another aspect of the present invention, the present invention provides a method for identifying chemical and/or biological substances that selectively kill or revert foxp3 and/or camta1 expressing regulatory T cells, comprising contacting one or more of said chemical and/or biological substance with said T cell, and detecting, whether said chemical and/or biological substance modulates the methylation of the CpG positions as analyzed and/or, optionally, opening of the chromatin structure in the region of the gene foxp3 and/or camta 1 or orthologous or paralogous genes thereof, and/or whether said one or more of said chemical and/or biological substance selectively kills (or inhibits) foxp3 and/or camta1 expressing regulatory T cells.

The method can be performed *in vitro* and/or *in vivo.* In this aspect, the present invention provides a method, sometimes called a "screening-method", that seeks to identify chemical and/or biological substances modulating foxp3 and/or camta1 expression that can be used as starting points for the development of regulatory T cell specific medication and respective pharmaceutical compositions. The present method is based on the fact that it is well accepted that the *foxp3* and/or *camta1* gene plays a central role for the development of regulatory T cells. Therefore, factors preventing Foxp3 and/or Camta1 expression are interesting for the treatment of tumour patients, where $Foxp3^+$ regulatory T cells have been shown to prevent a strong anti tumour response. Such factors, which lead to a stable modification of Foxp3 and/or Camta1 expression, can be detected with the method described in this invention.

Chemical and/or biological substances that are suitable as screening compounds are known to the person of skill and, for example, include small molecules, peptides and proteins, and antibodies or fragments thereof. Furthermore, the screening can be done using a commercially compound library, optimally together with suitable automation, such as a robot. In one preferred embodiment of the method for identifying chemical and/or biological substances, said substance provides a remethylation of the CpG positions as analyzed to at least 80%, preferably 90%, and more preferably 95%, and/or an closing of the chromatin structure in the region of the gene foxp3 and/or camta1 or orthologous or paralogous genes thereof.

In yet another aspect of the present invention, the present invention provides a kit for kit for diagnosing the survival of a cancer patient, comprising materials for performing a method according to the invention as above. The person of skill will furthermore be able to select materials for specific subsets of CpG positions in order to minimise the amount of sites to be analyzed, for example all sites as present on amplicon 1 or all sites as present on amplicon 2 or orthologous or paralogous CpG positions thereof. The same applies to the analysis of camta1. The kit can be a diagnostic kit.

The kits according to the present invention may also contain: 1. Chemicals (bisulfite, etc.) for processing the cell samples; 2. Procedure protocols; 3. Oligonucleotide probes, amplicons, blockers or extension primers according to the present invention that will detect markers relevant to a particular cell type. The oligonucleotides would be constructed to generate a signal on a commonly available detection platform, such as Real Time-PCR (RT-PCR) or Single Base Extension (SBE). Each signal indicates the level of methylation at a particular target site in the sample. As an alternative, probes according to the described nucleic acids could be produced for usage on a chip; 4. A bioinformatic tool to process the results. This, e.g., software might normalise the signals from the raw data, contain a result matrix for interpretation of the read-out, or implement various algorithms that calculate, for example, cell type proportions, or potency predictions, and 5. Materials to analyse the chromatin structure of the Foxp3 gene, in particular for ChIP-analysis.

Yet another preferred aspect of the present invention relates to an improved method of treatment of diseases that are related to Foxp3 and/or Camta1 expression, such as immunological diseases and/or tumorous diseases, in particular ovarian cancer, comprising a method as described herein above. The term "treatment" also includes a prevention of said Foxp3 and/or Camta1 expression related diseases.

The analysis of the methylation status and the chromatin packaging of the region within the *foxp3* and/or *camta1* loci allows an improved prediction whether the cell population will stably express the FoxP3 and/or Camta 1 gene or not.

Both Camta1 and the forkhead box transcription factor Foxp3 have been identified as a specific molecular marker for Tregs and its expression is essential for programming Treg development and function (for Foxp3, Fontenot, J.D. and Rudensky, A.Y. A well adapted regulatory contrivance: regulatory T cell development and the forkhead family transcription factor Foxp3. Nat Immunol 6, 331-7 (2005)). Although it is widely accepted that Foxp3+ Tregs represent a stable population mainly generated as a separate lineage in the thymus, conclusive data on the molecular mechanisms maintaining stable Foxp3 expression are not available. The inventors here provide first evidence that epigenetic modifications of the foxp3 and/or camta1 loci are required to enable long-term identity of $Foxp3^+$ and/or $Camta1^+$ Tregs.

The inventors have identified an element within the 5' untranslated region of the foxp3 locus, which displays demethylated CpG motifs both in developing thymic as well as in mature, peripheral Foxp3+CD25+CD4+ Tregs. Interestingly, a 374-bp stretch of the differentially methylated element is highly conserved between mice and humans (85.8% sequence identity), but not the region covered by amplicons 3 and 4, which essentially showed no signs of differential DNA methylation. Preliminary analyses using cells from human peripheral blood also showed a differential methylation of CpG motifs within the conserved element of the foxp3 locus when conventional CD25-CD4+ T cells and $CD25^{high}CD4+$ Tregs were compared, implying that this region and its epigenetic regulation is of functional importance. Similar considerations apply for Camta1.

In addition to DNA demethylation, also acetylated histones H3 and H4 as well as trimethylated histone H3 were associated with the conserved region in CD25+CD4+ Tregs, but not in conventional CD25-CD4+ T cells. Similar histone modifications have frequently been reported to concur with DNA demethylation, e.g. as described for the loci encoding the active cytokines IFN-γ and IL-4 in differentiated Th1 and Th2 cells, respectively (Reiner, S.L. Epigenetic control in the immune response. Hum Mol Genet 14 Spec No 1, R41-6 (2005), Tykocinski, L.O. et al. A critical control element for interleukin-4 memory expression in T helper lymphocytes. J Biol Chem 280, 28177-85 (2005)). These data suggest that in terminally differentiated Tregs epigenetic modifications of the foxp3 locus allow persistent expression of Foxp3. In silico analysis of the conserved region predicts a number of binding sites for transcription factors, including ATF/CREB, Elk-1, Ets-1, Evi-1, Foxp3, NFATc, NF-κB, SMAD-4 and STAT-1 (see Fig. 7), indicating that these factors might be involved in the induction of Foxp3 expression in CD25+CD4+ Tregs. Although the human FOXP3 promoter has recently been mapped to the putative 5' end of exon -2b, 6.1 kb upstream from the first coding exon6, other studies have reported promoter activity upstream from exon -1 close to the evolutionarily conserved region analyzed in the current study (Bassuny, W.M. et al. A functional polymorphism in the promoter/enhancer region of the FOXP3/Scurfin gene associated with type I diabetes. Immunogenetics 55, 149-56 (2003), Baratelli, F. et al. Prostaglandin E2 induces FOXP3 gene expression and T regulatory cell function in human CD4+ T cells. J Immunol 175, 1483-90 (2005)) corresponding to the Foxp3 mRNA species AY357712 and AY357713. Similar considerations apply for Camta1.

These data suggest that the evolutionarily conserved element might belong to an alternative TATA-less promoter, which contributes to the regulation of Foxp3 expression. The differential methylation status of the foxp3 locus in Tregs appears to be a new example for epigenetic regulation of cell lineage differentiation. Even though almost all cells in an individual contain the same complement of DNA code, higher organisms must impose and maintain different patterns of gene expression in the various types of differentiated cells. Most gene regulation is transitory, depending on the current state of the cell and changes in external stimuli. Persistent regulation, on the other hand, is a primary role of epigenetics - heritable regulatory patterns that do not alter the basic genetic coding of the DNA. DNA methylation is the archetypical form of epigenetic regulation; it serves as the stable memory for cells and performs a crucial role in maintaining the long-term identity of various cell types. The inventors' finding that evolutionarily conserved sequences within the foxp3 locus are completely and selectively demethylated upon differentiation in persistent Tregs suggests an important role of epigenetic fixation for this phenotype.

Moreover, this seems to be the first report that a transcription factor acting as master switch for a certain subpopulation is by itself subject to epigenetic control. The role of transcription factors such as T-bet or GATA-3 for the polarization of Th1 and Th2 cells, respectively, has been carefully studied and their interplay with epigenetically regulated regions in the respective cytokine genes is seen as a major factor in the acquisition of cytokine memory (Ansel, K.M., Lee, D.U. and Rao, A. An epigenetic view of helper T cell differentiation. Nat Immunol 4, 616-23 (2003), Reiner, S.L. Epigenetic control in the immune response. Hum Mol Genet 14 Spec No 1, R41-6 (2005)).

However, foxp3 appears to be the first known example, at least in the immune system, where the transcriptional regulation of a master transcription factor itself involves epigenetic mechanisms. Another example appears to be camta 1.

A crucial finding of this study is that chromatin remodelling of the foxp3 locus does not merely correlate with Foxp3 expression. Rather, the inventors' current data provide first experimental evidence that the completely demethylated status of the evolutionarily conserved region is only confined to stable Treg populations, such as the naturally occurring, thymus-derived CD25+CD4+ cells, and might indeed be a prerequisite for the permanent commitment to the suppressor cell lineage. This assumption is based on the analysis of TGF-β-induced Tregs, which in spite of Foxp3 expression and suppressive properties have not acquired a terminally differentiated phenotype and have lost Foxp3 expression upon restimulation in the absence of TGF-β This indicates that the recently postulated positive autoregulatory loop involving upregulation of endogenous TGF-β expression and subsequent Foxp3-dependent downregulation of Smad7, a negative regulator of TGF-β signalling, is not sufficient to induce stable Foxp3 expression in vitro (Fantini, M.C. et al. Cutting edge: TGF-beta induces a regulatory phenotype in CD4+CD25- T cells through Foxp3 induction and down-regulation of Smad7. J Immunol 172, 5149-53 (2004)). As TGF-β-induced Tregs display only weakly demethylated CpG motifs within the conserved region of the foxp3 locus, a more complete CpG demethylation might be the key for a stable Foxp3 expression, similar to what has recently been reported for IL-2 (Bruniquel, D. and Schwartz, R.H. Selective, stable demethylation of the interleukin-2 gene enhances transcription by an active process. Nat Immunol 4, 235-40 (2003)).

The findings of this study have important implications with respect to clinical applications, as the detection of demethylated foxp3 and/or camta1 sequences might allow the development of novel diagnostic tools for the quantification of Tregs in blood or tissues.

An increased number of Tregs was observed in patients with a variety of malignant cancers (Schaefer, C. et al. Characteristics of CD4+CD25+ regulatory T cells in the peripheral circulation of patients with head and neck cancer. Br J Cancer 92, 913-20 (2005), Ormandy, L.A. et al. Increased populations of regulatory T cells in peripheral blood of patients with hepatocellular carcinoma. Cancer Res 65, 2457-64 (2005), Roncador, G. et al. FOXP3, a selective marker for a subset of adult T-cell leukaemia/lymphoma. Leukemia 19, 2247-53 (2005)) and is involved in tumour progression (Curiel, T.J. et al. Specific recruitment of regulatory T cells in ovarian carcinoma fosters immune privilege and predicts reduced survival. Nat Med 10, 942-9 (2004), Wolf, D. et al. The expression of the regulatory T cell-specific forkhead box transcription factor FoxP3 is associated with poor prognosis in ovarian cancer. Clin Cancer Res 11, 8326-31 (2005), Beyer, M. et al. In vivo peripheral expansion of naive CD4+CD25high FOXP3+ regulatory T cells in patients with multiple myeloma. Blood (2006)).

Most notably, the presence of Tregs, as defined by the gene expression of FOXP3, has been shown to constitute a significant predictive parameter for the clinical outcome in ovarian cancer patients (Curiel, T.J. et al. Specific recruitment of regulatory T cells in ovarian carcinoma fosters immune privilege and predicts reduced survival. Nat Med 10, 942-9 (2004), Wolf, D. et al. The expression of the regulatory T cell-specific forkhead box transcription factor FoxP3 is associated with poor prognosis in ovarian cancer. Clin Cancer Res 11, 8326-31 (2005)). However, the analytical value of flow cytometry, immunohistological and mRNA expression analysis of CD25 and Foxp3 as an accurate diagnostic tool is blurred by both the ambiguity of the markers and the instability of the biological materials.

Measurement of the methylation status of the foxp3 and/or camta1 loci could present more reliable and objective criteria for the identification and quantification of Tregs. Moreover, DNA methylation is intrinsically a more stable parameter than mRNA expression or protein synthesis and can be accurately quantified (Baron, U. et al. DNA Methylation Analysis as a Tool for Cell Typing. Epigenetics 1, 55-60 (2006)). Therefore, the inventors believe that establishment of a measurement system for the methylation status of the human foxp3 and/or camta1 loci provides a novel diagnostic tool in tumour patients.

The present invention shall now be further described in the following examples without being limited thereto. It should be noted that the present examples have been performed with foxp3, nevertheless, the person of skill will be readily able to modify the examples in order to adopt these to camta1-analysis. In the accompanying Figures and Sequences,

SEQ ID No. 1 to SEQ ID No. 10 show preferred oligonucleotides as used in the present examples for foxp3.

SEQ ID No. 11 shows the nucleic acid sequence of camta1.

Table 1 shows the methylation status of individual CpG motifs within the foxp3 locus. Summary of all DNA methylation analyses performed for indicated subsets. Shown is the degree of methylation (in %) for individual CpG motifs (n.a., not analyzed). CpG motifs from amplicon 2 overlapping with motifs in amplicon 1 were excluded.

Figure 1 shows the stable Foxp3 expression in CD25+CD4+ Tregs. CD25+CD4+ Tregs were isolated ex vivo, labelled with CFSE and transferred into syngenic recipients ($2x10^6$/mouse). Before transfer, sorted cells were analyzed for intracellular Foxp3 expression by FACS. Fourteen days after transfer, splenocytes of recipient mice were stained for CD4, CD25 and Foxp3 and analyzed by FACS. Representative dot and histogram plots from four independently analyzed mice were selected. Numbers display frequency of cells within indicated populations. Comparable results were obtained for cells isolated from peripheral or mesenteric LNs.

Figure 2 shows the selectively demethylated CpG motifs within the foxp3 locus in CD25+CD4+ Tregs isolated from secondary lymphoid organs. (A) Schematic view on the foxp3 locus depicts exon-intron structure and position of selected amplicons (Amp 1-4). Shown is the distribution and position of individual CpG motifs within the amplicons. (B) CD25+CD4+ and CD25-CD4+ T cells were sorted from spleens and lymph nodes pooled from 20 male BALB/c mice. FACS analysis shows the sort purity (upper panel) and Foxp3 expression in sorted subsets (lower panel). Numbers display frequency of cells within indicated populations. (C) Methylation pattern of selected amplicons of the foxp3 locus in CD25+CD4+ Tregs and conventional CD25-CD4+ T cells. The amplicons are subdivided by horizontal lines each representing an individual CpG motif. ESME software is used for normalisation and quantification of methylation signals from sequencing data by calculating ratios of T and C signals at CpG sites. Data are condensed to methylation information at CpG positions forming matrices of consecutive CpGs. The methylation status of individual CpG motifs within the four amplicons is gray-coded according to the degree of methylation at that site. The color code ranges from yellow (0% methylation) to blue (100% methylation) according to the gray scale on the right. CpG motifs from amplicon 2 overlapping with motifs in amplicon 1 were excluded. Due to sequencing problems, the CpG motif 37 from amplicon 4 is not listed. One representative out of two individual experiments is shown.

Figure 3 shows the increased histone acetylation and K4 trimethylation in CD25+CD4+ Tregs. ChIP assays were performed with CD25+CD4+ Tregs (black bars) and conventional CD25-CD4+ T cells (open bars) sorted from spleens and lymph nodes pooled from 20 male BALB/c mice. DNA fragments binding to acetylated or trimethylated histones were immunoprecipitated using antibodies directed against acetylated histone H3, acetylated histone H4 or trimethylated histone H3 at position K4. A rabbit isotype IgG served as control. Precipitated DNA was quantified by real-time PCR with primers specific for the differentially methylated region of the foxp3 locus. Sample PCR products were set in relation to input DNA. One representative out of two individual experiments is shown.

Figure 4 shows the demethylated CpG motifs within the foxp3 locus in CD25+ CD4 SP thymocytes. (A) CD25+CD4+CD8- and CD25-CD4+CD8- subsets were sorted from thymocytes pooled from 30 male BALB/c mice. FACS analysis shows purity of sorted subsets (upper panel) and Foxp3 expression in gated CD25- and CD25+ subsets of CD4+ SP thymocytes (lower panel). Numbers display frequency of cells within indicated populations. (B) Methylation pattern of selected amplicons of the foxp3 locus in CD25+ and CD25- CD4 SP thymocytes. The methylation status of individual CpG motifs within the four amplicons is color-coded as described in Figure 2. One representative out of two individual experiments is shown.

Figure 5 shows that TGF-β-induced Tregs harbour partially demethylated CpG motifs within the foxp3 locus. (A) CD25-CD4+ T cells sorted from spleens and lymph nodes pooled from 20 male BALB/c mice were cultured for 6 days in the presence of TGF-β. Cells cultured under Th1 conditions were used as control. Within the starting population, <1% of the cells were Foxp3+ (not shown). On day 6, cultured cells were FACS-sorted for CD25+ cells. FACS analysis shows the purity of sorted subsets (upper panel) and Foxp3 expression in gated CD25+ cells derived from indicated cultures (lower panel). Numbers display frequency of cells within indicated populations. (B) Methylation pattern of selected amplicons of the foxp3 locus in sorted CD25+ cells derived from indicated cultures. The methylation status of individual CpG motifs within the four amplicons is color-coded as described in Figure 2 (n.a., not analyzed). One representative out of two individual experiments is shown.

Figure 6 shows that TGF-β-induced Foxp3+ Tregs loose Foxp3 expression upon restimulation. (A) CD25-CD4+ T cells were cultured for 6 days in the presence of TGF-β as described in Figure 5. On day 6, cultured cells were FACS-sorted for CD25+ cells and analyzed for intracellular Foxp3 expression by FACS (upper panel). Sorted CD25+ cells from the same TGF-□-cultures were restimulated for another 6 days in the absence of TGF-β. On day 6, cultured cells were analyzed for intracellular Foxp3 expression by FACS (lower panel). Numbers display frequency of cells within indicated populations. (B) Ex vivo isolated CD25+CD4+ Tregs were analyzed for intracellular Foxp3 expression by FACS (upper panel) and expanded by in vitro stimulation for 6 days. On day 6, expanded Tregs were analyzed for intracellular Foxp3 expression by FACS (lower panel). Numbers display frequency of cells within indicated populations. One representative out of two individual experiments is shown.

Figure 7 shows CpG motifs and transcription factor binding sites within conserved region of the foxp3 locus. Alignment of mouse (upper row) and human (lower row) genomic sequences corresponding to amplicons 1 and 2 of the foxp3 locus identified a highly conserved 374-bp region, which is depicted in bold letters. Individual CpG motifs are shown underlined and putative transcription factor binding site core elements, which were identified using the TRANSFAC® database and the search tool MATCH®, are illustrated in bold.

Figure 8 shows the differential methylation of the gene for CAMTA1 between regulatory T-cells and 9 types of blood cells. The basis for the gene is CAMTA1 : Calmodulin-binding transcription activator 1; Ensembl Human Gene: ENSG00000171735. The abbreviations for the types of blood cells are as follows: TREG CD4+ CD25+ FOXP3+ regulatory T-cells; BCST18 CD15+ granulocytes; BCST19 CD14+ monocytes; BCST20 CD56+ NK-cells; BCST21 CD4+CD27+CD45RA+ naive T-cells; BCST22 CD4+CD27+CD45RA- memory T-cells; BCST23 CD8+CD27+CD45RA+ naive cytotoxic T-cells; BCST24 CD8+CD27+CD45RA-memory cytotoxic T-cells; BCST25 CD19+ naive B-cells; and BCST26 memory B-cells. On the left side, the CpG-positions of the amplicon as analysed (amplicon 869) are indicated.

## Examples

### Mice

BALB/c mice were bred at the BfR (Bundesinstitut fuer Risikobewertung, Berlin, Germany) and used at 6-12 wk of age. All animal experiments were performed under specific pathogen free conditions and in accordance with institutional, state and federal guidelines.

### Antibodies, staining reagents and cytokines

The following antibodies were produced in the inventors' laboratory: anti FcR II/III (2.4G2), anti CD4 (GK1.5), anti CD3 (145.2C11), anti CD28 (37.51) and anti IL-4 (11B11). The following antibodies and secondary reagents were purchased from BD PharMingen: anti CD4 (RM4-5), anti CD 19 (1D3), anti CD25 (7D4), anti CD8 (53-6.7), anti CD25 (PC6.1), anti CD62L (Mel-14), streptavidin, and appropriate isotype controls. The PE anti-mouse Foxp3 staining set was purchased from eBioscience. All microbeads were obtained from Miltenyi Biotec and all cytokines from RandD systems.

### Flow cytometry

Cytometric analysis was performed as previously described (Huehn, J. et al. Developmental stage, phenotype and migration distinguish naive- and effector/memory-like CD4+ regulatory T cells. J Exp Med 199, 303-313 (2004)) using a FACS Calibur or a LSRII (BD Biosciences) and the CellQuest™ software. Dead cells were excluded by PI (propidium iodide) or DAPI (diamidophenylindole) staining (Sigma). Intracellular Foxp3 staining was performed with the PE anti-mouse Foxp3 staining set according to the manufacturers instructions.

Ex vivo cell sorting

CD4+ T cells were isolated from pooled spleen and lymph node (LN) single cell suspensions by using anti CD4-FITC plus anti FITC multisort microbeads and the AutoMACS magnetic separation system (Miltenyi Biotec). After release of beads according to the manufacturer's instructions, CD25+ and CD25- cells were separated using anti CD25-APC plus anti APC microbeads. Thymic single cell suspensions were sorted for CD8+ and CD8- cells using anti CD8 microbeads and the AutoMACS magnetic separation system. MACS-sorted CD8-thymocytes were subsequently stained using anti CD4-FITC, anti CD25-APC and anti CD19-PE and sorted into CD25+ and CD25- subsets of CD4 SP thymocytes as well as into CD19-DN thymocytes by FACS (Aria, BD Bioscience). MACS-sorted CD8+ thymocytes were stained using anti CD4-FITC and anti CD8-PerCP and sorted into DP thymocytes by FACS. All subsets were sorted to a purity of >98%.

Adoptive transfer of sorted CD25+CD4+ T cells

Sorted CD25+CD4+ T cells were labeled with CFSE (Molecular Probes) as described before (Siegmund, K. et al. Migration matters: regulatory T cell compartmentalization determines suppressive activity in vivo. Blood 106, 3097-3104 (2005)). 2 x $10^6$ CFSE-labeled cells were adoptively transferred into syngenic recipients. Fourteen days after transfer, single cell suspensions of spleen, peripheral and mesenteric LNs were prepared and stained for CD4, CD25 and Foxp3 as described above.

TGF-β-induced Tregs

CD4+ T cells were isolated from pooled spleen and LN single cell suspensions by using anti CD4-FITC plus anti FITC multisort microbeads and the AutoMACS magnetic separation system (Miltenyi Biotec). After release of beads according to the manufacturer's instructions, CD25+ cells were depleted by using anti CD25-APC plus anti APC microbeads. To avoid the expansion of pre-committed Foxp3+ Tregs, the inventors excluded the majority of residual Foxp3+ Tregs from the CD25-CD4+ T cell fraction by sorting for CD62L$^{high}$ cells using anti CD62L microbeads. MACS-sorted CD62L$^{high}$CD25-CD4+ T cells were stimulated for 3 days using plate bound anti CD3 (6 μg/ml) and anti CD28 (4 μg/ml). For Th1 cultures 5 μg/ml anti-IL-4, 20 ng/ml IFN-γ and 5 ng/ml IL-12 was added to the medium. For TGF-β cultures 5 ng/ml TGF-β and 10 ng/ml IL-2 was used. After 3 days, cells were removed from the stimulus, transferred to non-coated plates and cultured for another 3 days. All cell culture was done with RPMI 1640 (Gibco) supplemented with 10% FCS (Sigma). On day 6, cultured cells were stained using anti CD25-APC and sorted for CD25+ cells by FACS (Aria). Foxp3 expression of sorted CD25+ cells was analyzed by intracellular staining. For restimulation experiments sorted CD25+ cells were stimulated for 3 days using plate bound anti CD3 (6 μg/ml) and anti CD28 (4 μg/ml) plus IL-2 (10 ng/ml). After 3 days, cells were removed from the stimulus, transferred to non-coated plates and cultured for another 3 days. On day 6, cultured cells were stained for CD25 and Foxp3 as described above.

Expansion of CD25+CD4+ Tregs

CD25+ cells were enriched from pooled spleen and LN single cell suspensions by using anti CD25-FITC, anti FITC microbeads and the AutoMACS magnetic separation system (Miltenyi Biotec). MACS-enriched CD25+ T cells were subsequently stained using anti CD4-PerCP and anti CD62L-APC and sorted for CD62L$^{high}$CD25+CD4+ Tregs by FACS (Aria, BD Bioscience). CD62L$^{high}$ Tregs were used to avoid the expansion of Foxp3-CD25+ effector T cells. FACS-sorted CD62L$^{high}$CD25+CD4+ Tregs were stimulated for 3 days using plate bound anti CD3 (6 μg/ml) and anti CD28 (4 μg/ml) plus IL-2 (40 ng/ml) followed by transfer to non-coated plates and culture for another 3 days. On day 6, cultured cells were stained for CD25 and Foxp3 as described above.

Bisulphite sequencing

Genomic DNA was isolated from purified T cells using the DNeasy tissue kit (Qiagen) following the supplier's recommendations. Sodium bisulphite treatment of genomic DNA was performed according to Olek et al. (Olek, A., Oswald, J. and Walter, J. A modified and improved method for bisulphite based cytosine methylation analysis. Nucleic Acids Res 24, 5064-6 (1996)) with minor modifications, resulting in the deamination of unmethylated cytosines to uracil, whereas methylated cytosines remain unchanged. In a subsequent PCR amplification uracils were replicated as thymidines. Thus, detection of a "C" in sequencing reactions reflects methylation of the genomic DNA at that site. Detection of a "T" at the same site instead reflects the absence of a methyl modification of the genomic cytosine.

PCRs were performed on MJ Research thermocyclers (Waltham) in a final volume of 25 μl containing 1 x PCR Buffer, 1 U Taq DNA polymerase (Qiagen), 200 μM dNTPs, 12.5 pmol each of forward and reverse primers, and 7 ng of bisulphite-treated genomic DNA. The amplification conditions were 95°C for 15 min and 40 cycles of 95°C for 1 min, 55°C for 45 sec and 72°C for 1 min and a final extension step of 10 min at 72°C. PCR products were purified using ExoSAP-IT USB Corp.) and sequenced in both directions applying the PCR primers and the ABI Big Dye Terminator v1.1 cycle sequencing chemistry (Applied Biosystems) followed by capillary electrophoresis on an ABI 3100 genetic analyzer. Trace files were interpreted using ESME, which normalizes sequence traces, corrects for incomplete bisulphite conversion and allows for quantification of methylation signals (Lewin, J., Schmitt, A.O., Adorjan, P., Hildmann, T. and Piepenbrock, C. Quantitative DNA methylation analysis based on four-dye trace data from direct sequencing of PCR amplificates. Bioinformatics 20, 3005-12 (2004)). For each sample both PCR amplification and sequencing was repeated once. The following primers (5' to 3' direction) were used for both PCR amplification of bisulphite converted genomic DNA and sequence reactions:

Amp 1 (fw: AGGAAGAGAAGGGGGTAGATA; SEQ ID No. 1; rev: AAACTAACATTCCAAAACCAAC; SEQ ID No. 2)

Amp 2 (fw: ATTTGAATTGGATATGGTTTGT; SEQ ID No. 3; rev: AACCTTAAACCCCTCTAACATC; SEQ ID No. 4)

Amp 3 (fw: AGAGGTTGAAGGAGGAGTATTT; SEQ ID No. 5; rev: ACTATCTATCCAATTCCCCAAC; SEQ ID No. 6)

Amp 4 (fw: TGGTTGTTTTGGAGTTTAGTGT; SEQ ID No. 7; rev: CACTTTTCTACCTTCCACAAAT; SEQ ID No. 8)

Chromatin immunoprecipitation (ChIP)

ChIP analysis was carried out according to the manufacturer's protocol (Upstate). Cells (1-5 x $10^6$) were fixed with 1 % formaldehyde and chromatin was fragmented by ultrasound. For all ChIP samples, sheared chromatin was precleared by incubation with ProteinA/agarose/salmon sperm DNA (Upstate). Subsequently, chromatin was immunoprecipitated by overnight incubation at 4°C with 4 μg antibodies (rabbit isotype, #2027, Santa Cruz; anti acetyl-histone H3, #06-599, Upstate; anti acetyl-histone H4, #06-866, Upstate; anti trimethyl-K4-histone H3, #07-473, Upstate) followed by incubation with Protein A-agarose/salmon sperm DNA for 1h. Precipitates were defixed and DNA was purified by using the NucleoSpin® Extract II kit (Macherey-Nagel). The amount of immunoprecipitated DNA was quantified by real-time PCR with LightCycler (Roche Applied Science) using SYBR Green and the following primer pair (5' to 3' direction):

Foxp3 (331 bp) fw: GACTCAAGGGGGTCTCA; SEQ ID No. 9; rev: TTGGGCTTCATCGGCAA; SEQ ID No. 10.

Sample PCR products were set in relation to the input DNA using the following equation:

$$E\char`^([DNA_{input}]-[DNA_{IP}]).$$

Bioinformatics

Genomic sequences spanning the foxp3 locus were analyzed using the alignment software vista (http://pipeline.lbl.gov/servlet/vgb2/), allowing the identification of conserved regions. Transcription factor binding sites were identified using the TRANSFAC® database (Matys, V. et al. TRANSFAC and its module TRANSCompel: transcriptional gene regulation in eukaryotes. Nucleic Acids Res 34, D 108-10 (2006)) and the search tool MATCH® (Kel, A.E. et al. MATCH: A tool for searching transcription factor binding sites in DNA sequences. Nucleic Acids Res 31, 3576-9 (2003)).

CD25+CD4+ Tregs display a stable Foxp3 expression

It is assumed that Foxp3+CD25+CD4+ Tregs represent an individual lineage exhibiting a stable phenotype. To prove experimentally the stability of Foxp3 expression in natural Tregs on a cellular level, the inventors adoptively transferred sorted CD25+CD4+ T cells after CFSE-labeling into syngeneic recipients. Fourteen days after transfer, >95% of CFSE+ cells, including those which had divided once or twice according to loss of CFSE, were still Foxp3+ (Figure 1), supporting data from a recent publication using a lymphopenic transfer model (Suffia, I.J., Reckling, S.K., Piccirillo, C.A., Goldszmid, R.S. and Belkaid, Y. Infected site-restricted Foxp3+ natural regulatory T cells are specific for microbial antigens. J Exp Med 203, 777-88 (2006)). Having shown that Foxp3 is stably expressed in CD25+CD4+ Tregs, the inventors next asked whether epigenetic modifications of the foxp3 locus might account for the maintenance of the long-term identity of Foxp3+ Tregs.

Epigenetic modifications of the foxp3 locus in Foxp3+CD25+CD4+ Tregs

The lymphoproliferative disorder of scurfy mice is completely rescued by transgenic complementation with a 30.8-kb genomic fragment containing the foxp3 gene from wild type mice (Brunkow, M.E. et al. Disruption of a new forkhead/winged-helix protein, scurfin, results in the fatal lymphoproliferative disorder of the scurfy mouse. Nat Genet 27, 68-73 (2001)). This indicates that all regulatory elements required for proper Foxp3 expression are located within the transgene including the entire gene, as well as 12.5 kb and 2.8 kb of 5' and 3' flanking sequences, respectively. The inventors therefore focused the inventors' analysis of epigenetic modifications on sequences from the 30.8-kb region and selected specific regions for methylation analysis based on CpG density (Figure 2A): Overlapping amplicons 1 and 2 map upstream from exon -1, amplicon 3 and 4 align to the 7th intron. No CpG-rich regions were observed within the Foxp3 promoter located at the putative 5' end of exon -2b, 6.1 kb upstream from the first coding exon (Mantel, P.Y. et al. Molecular Mechanisms Underlying FOXP3 Induction in Human T Cells. J Immunol 176, 3593-602 (2006), Brunkow, M.E. et al. Disruption of a new forkhead/winged-helix protein, scurfin, results in the fatal lymphoproliferative disorder of the scurfy mouse. Nat Genet 27, 68-73 (2001)).

The inventors sorted CD25+CD4+ Tregs and conventional CD25-CD4+ T cells from secondary lymphoid organs of male mice. Male mice were chosen to avoid potential artifacts due to random X chromosome inactivation since Foxp3 is encoded on the X-chromosome (Brunkow, M.E. et al. Disruption of a new forkhead/winged-helix protein, scurfin, results in the fatal lymphoproliferative disorder of the scurfy mouse. Nat Genet 27, 68-73 (2001)). As expected, the vast majority of sorted CD25+CD4+ Tregs were Foxp3+, whereas <1% of CD25-CD4+ T cells expressed Foxp3 (Figure 2B).

The methylation status of the foxp3 locus was analyzed by bisulphite sequencing (see Methods). Interestingly, striking differences between CD25+CD4+ Tregs and conventional CD25-CD4+ T cells could be observed. CpG motifs within amplicons 1 and 2 displayed a high degree of methylation (~100%) within conventional CD25-CD4+ T cells, but were almost completely demethylated within CD25+CD4+ Tregs (Figure 2C and see Supplementary Table 1 online). No significant differences were observed for amplicons 3 and 4, showing that the demethylation process is not a random event, but is confined to defined regions as was recently found for the IL-2 promoter ( Bruniquel, D. and Schwartz, R.H. Selective, stable demethylation of the interleukin-2 gene enhances transcription by an active process. Nat Immunol 4, 235-40 (2003)). Together, the inventors' findings suggest that demethylation of CpG motifs within selected elements of the foxp3 locus enable stable Foxp3 expression in CD25+CD4+ Tregs.

Histone modifications indicate opening of the foxp3 locus in CD25+CD4+ Tregs

DNA demethylation is often linked to acetylation or methylation of histones, other key features of chromatin remodelling (Dobosy, J.R. and Selker, E.U. Emerging connections between DNA methylation and histone acetylation. Cell Mol Life Sci 58, 721-7 (2001)). To investigate whether this also holds true for the aforementioned region of the foxp3 locus, the inventors performed chromatin immunoprecipitation (ChIP) experiments using antibodies specific for acetylated histone H3, acetylated histone H4 and trimethylated lysine 4 of histone H3 (H3K4). Subsequently, the precipitated DNA was used as a template for amplifying the differentially methylated region of the foxp3 locus by quantitative real-time PCR. Indeed, in CD25+CD4+ Tregs the region of interest showed a stronger association with modified histones when compared with conventional CD25-CD4+ T cells (Figure 3). Major differences were observed for the acetylated and trimethylated histone H3, whereas minor differences were found for the acetylated histone H4. Together, the ChIP data disclose that within conventional CD25-CD4+ T cells the foxp3 locus is packed in a more condensed, inaccessible chromatin structure whereas it is located within open euchromatin in CD25+CD4+ Tregs.

Demethylated CpG motifs in developing Foxp3+ thymocytes

Having shown that peripheral CD25+CD4+ Tregs display a characteristic methylation status of the foxp3 locus, the inventors next sought to determine whether this also could be observed in developing Tregs. Generation of Foxp3+ cells in the thymus occurs preferentially at the CD4 SP stage or during transition to this stage (Fontenot, J.D., Dooley, J.L., Farr, A.G. and Rudensky, A.Y. Developmental regulation of Foxp3 expression during ontogeny. J Exp Med 202, 901-6 (2005)). The inventors therefore isolated CD25+ and CD25- subsets of CD4 SP thymocytes from male mice. As expected, ~80% of CD25+ CD4 SP thymocytes were Foxp3+, whereas <1% of CD25- CD4 SP thymocytes expressed Foxp3 (Figure 4A). Only CD25+ but not CD25- CD4 SP thymocytes displayed demethylated CpG motifs within the regions covered by amplicons 1 and 2, whereas CpG motifs in amplicons 3 and 4 were again fully methylated in both subsets (Figure 4B and see Table 1). As expected, in double-negative (DN) and double-positive (DP) thymocytes, which show hardly any Foxp3 expression (Fontenot, J.D. et al. Regulatory T cell lineage specification by the forkhead transcription factor foxp3. Immunity 22, 329-41 (2005), Fontenot, J.D., Dooley, J.L., Farr, A.G. and Rudensky, A.Y. Developmental regulation of Foxp3 expression during ontogeny. J Exp Med 202, 901-6 (2005)), CpG motifs within the regions covered by amplicons 1 and 2 were completely methylated (see Table 1), supporting the inventors' assumption that Foxp3 expression is developmentally regulated and requires an opening of the foxp3 locus.

When compared to peripheral CD25+CD4+ Tregs, in which the CpG motifs within amplicons 1 and 2 were almost completely demethylated (mean degree of methylation <3%), CD25+ CD4 SP thymocytes showed a clearly reduced degree of demethylated DNA (mean degree of methylation ~50%) with individual CpG motifs being even completely methylated (see Table 1). These stark differences cannot simply be explained by the fact that, among CD25+ CD4 SP thymocytes, only 80% are Foxp3+ compared to 95% within the peripheral counterpart (Figure 2B and 4A). Rather it indicates that the locus does not become fully opened until completion of maturation and exit from the thymus.

Weak CpG demethylation correlates with poor Foxp3 stability in TGF-β-induced Tregs

A critical issue for application of Tregs in therapeutic approaches is the availability of large numbers of cells. Recent publications have reported that conventional CD25-CD4+ T cells can be converted into Foxp3+ Tregs by stimulation in the presence of TGF-β (Chen, W. et al. Conversion of peripheral CD4+CD25- naive T cells to CD4+CD25+ regulatory T cells by TGF-beta induction of transcription factor Foxp3. J Exp Med 198, 1875-86 (2003), Park, H.B., Paik, D.J., Jang, E., Hong, S. and Youn, J. Acquisition of anergic and suppressive activities in transforming growth factor-beta-costimulated CD4+CD25-T cells. Int Immunol 16, 1203-13 (2004), Fu, S. et al. TGF-beta induces Foxp3 + T-regulatory cells from CD4 + CD25 - precursors. Am J Transplant 4, 1614-27 (2004), Fantini, M.C. et al. Cutting edge: TGF-beta induces a regulatory phenotype in CD4+CD25- T cells through Foxp3 induction and down-regulation of Smad7. J Immunol 172, 5149-53 (2004), Wan, Y.Y. and Flavell, R.A. Identifying Foxp3-expressing suppressor T cells with a bicistronic reporter. Proc Natl Acad Sci U S A 102, 5126-31 (2005), Fantini, M.C. et al. TGF-{beta} induced Foxp3+ regulatory T cells suppress Th1-mediated experimental colitis. Gut (2005)). However, the stability and in vivo efficacy of these cells have not been thoroughly tested so far. Analysis of the accessibility of the foxp3 locus might provide an additional clue, aside from the mere expression of Foxp3, as to the extent to which a permanent conversion into a Treg lineage did occur. The inventors therefore analyzed the methylation status of the foxp3 locus from CD25-CD4+ T cells, which had been activated and cultured for 6 days in the presence of TGF-β. On day 6, >98% of TGF-β-cultured cells were Foxp3+, whereas control cells cultured under Th1 conditions showed only ~1% Foxp3 expression (Figure 5A).

As expected, within cultured Th1 cells all analyzed CpG motifs were completely methylated (Figure 5B and Table 1). In contrast, cell culture in the presence of TGF-β led to a clearly visible demethylation of CpG motifs within the region covered by amplicons 1 and 2, whereas CpG motifs in amplicons 3 and 4 again were fully methylated. However, the degree of demethylation was far less pronounced compared to naturally occuring, peripheral CD25+CD4+ Tregs (Figure 2C). This prompted us to investigate whether such a weak degree of CpG demethylation might correlate with persistent expression of Foxp3 in TGF-β-induced Tregs. Therefore, the inventors restimulated TGF-β-cultured Foxp3+ cells for another 6 days in the absence of TGF-β followed by the analysis of intracellular Foxp3 expression. As a control, the inventors cultured ex vivo isolated Foxp3+CD25+CD4+ Tregs under comparable conditions. Whereas ex vivo CD25+CD4+ Tregs maintained high Foxp3 levels, the vast majority of TGF-β-induced Tregs have lost Foxp3 expression during the 6-day restimulation period (Figure 6). Both cultures displayed comparable proliferation rates, making selective outgrowth of Foxp3- cells from the TGF-β-cultured cells unlikely. Viewed as a whole, the inventors' data strongly suggest that complete demethylation of CpG motifs within the foxp3 locus is required to allow for a stable Foxp3 expression.

## Claims

**1.** A method for pan-cancer diagnostics, comprising identifying the amount and/or proportion of stable regulatory T cells in a patient suspected of having cancer through analysing the methylation status of at least one CpG position in the gene foxp3 and/or camta1 or orthologous or paralogous genes thereof, wherein an increased amount and/or proportion of stable regulatory T cells in said patient is indicative for an unspecific cancerous disease.

**2.** The method according to claim 1, further comprising the analysis of at least one cancer- and/or tissue-specific marker.

**3.** A method for diagnosing the survival of a cancer patient, comprising
identifying the amount and/or proportion of stable regulatory T cells in said cancer patient through analysing the methylation status of at least one CpG position in the gene foxp3 and/or camta1 or orthologous or paralogous genes thereof,
wherein a demethylation in the gene foxp3 and/or camta1 or orthologous or paralogous genes thereof, is indicative of a stable regulatory T cell, and
wherein an increased amount and/or proportion of stable regulatory T cells in said cancer patient is indicative for a shorter survival for said cancer patient.

**4.** The method according to any of claims 1 to 3, wherein said stable regulatory T cell is a $CD25^+CD4^+$ regulatory T cell.

**5.** The method according to any of claims 1 to 4, further comprising the step of analysing the packaging of the chromatin structure in the region of the gene foxp3 and/or camta1 or orthologous or paralogous genes thereof, wherein an open chromatin structure in the region of the gene foxp3 and/or camta1 or orthologous or paralogous genes thereof is indicative of a stable regulatory T cell.

**6.** The method according to any of claims 1 to 5, wherein said analysis of the methylation status comprises analysing the methylation status of at least one CpG position in the 5' region upstream from the transcription start, promoter regions, introns, and/or exon/intron borders of the gene foxp3 and/or camta1.

**7.** The method according to any of claims 1 to 6, wherein said analysis of the methylation status of foxp3 comprises amplification with at least one of the primer pairs selected from SEQ ID No. 1 and 2; SEQ ID No. 3 and 4, and orthologous or paralogous primer pairs thereof.

**8.** The method according to any of claims 1 to 7, wherein said analysis of the methylation status comprises a method selected from methylation specific enzymatic digests, bisulphite sequencing, MSP, HeavyMethyl, MethyLight, Ms-SNuPE or other methods relying on a detection of amplified DNA.

**9.** The method according to any of claims 1 to 8, wherein the analysis of the packaging of the chromatin structure comprises chromatin immunoprecipitation, in particular using antibodies against acetylated histones, in particular H3 and/or H4.

**10.** The method according to any of claims 1 to 9, wherein said patient is a human.

**11.** The method according to any of claims 1 to 10, wherein said cancer is selected from solid tumors, breast cancer, ovarian cancer, prostate cancer, and lung cancer.

**12.** The method according to any of claims 1 to 11, wherein the amount and/or proportion of stable regulatory T cells is identified in a blood or tissue sample obtained from said human.

**13.** The method according to any of claims 1 to 12, wherein the amount and/or proportion of stable regulatory T cells is identified through a comparison with the methylation status of at least one CpG position in the gene foxp3 and/or camta1 or orthologous or paralogous genes thereof in samples selected from all cells as present in said sample, all T-cells as present in said sample, and the number of stable regulatory T cells in a healthy patient.

**14.** The method according to any of claims 1 to 13, wherein an amount of regulatory T-cells corresponds to a demethylation of the CpG positions as analyzed to at least 80%, preferably 90%, and more preferably 95%.

**15.** The method according to any of claims 1 to 14, wherein a demethylation and/or open chromatin structure in the region of the gene foxp3 and/or camta1 or orthologous or paralogous genes thereof corresponds to at least 10-fold of DNA relative to input for $Me_3K4$.

**16.** The method according to any of claims 1 to 15, further comprising the step of providing a treatment for said cancer patient wherein said treatment reduces the amount and/or proportion of stable regulatory T cells in said cancer patient.

**17.** The method according to claim 16, wherein said treatment is selected from providing chemical and/or biological substances that selectively kill regulatory T-cells in said patient, or a treatment that reduces the expression of the gene foxp3 and/or camta1 or inhibits the biological activity of FoxP3 and/or Camta1 in said regulatory T-cells in said patient.

**18.** The method according to claim 16 or 17, wherein said substance is selected from an antibody that is selective for regulatory T-cells, such as an anti-CD25-antibody, a cytotoxic substance that is selective for regulatory T-cells, such as denileukin diftitox (Ontak®), antisense nucleic acids against the expression of the gene foxp3, and methylating agents.

**19.** The method according to any of claims 16 to 18, further comprising measuring and/or monitoring the amount of said regulatory T cells in response to chemical and/or biological substance.

**20.** A kit for diagnosing the survival of a cancer patient, comprising materials for performing a method according to any of claims 1 to 19.

Table 1

| | periphery | | | | thymus | | | | | | *in vitro* | | |
|---|---|---|---|---|---|---|---|---|---|---|---|---|---|
| | | | | | CD4SP | | | | DP | DN | | | |
| amplicon (CpG position) | CD25$^-$ | | CD25$^+$ | | CD25$^-$ | | CD25$^+$ | | | | Th1 | TGF-β | |
| | exp. 1 | exp. 2 | exp. 1 | exp. 2 | exp. 1 | exp. 2 | exp. 1 | exp.2 | | | | ex . 1 | exp.2 |
| 1 (158) | 100 | 100 | 4,5 | 0 | 100 | 100 | 29 | 44 | 100 | 100 | 100 | 100 | 100 |
| 1 (180) | 100 | 100 | 3.5 | 12 | 100 | 100 | 40.5 | 47 | 100 | 100 | 100 | 92 | 93. |
| 1 (308) | 100 | 100 | 0 | 0 | 100 | 100 | 20 | 33 | 100 | 100 | 100 | 63 | 75 |
| 1 (344) | 100 | 100 | 0 | 0 | 100 | 100 | 38.5 | 37.5 | 100 | 100 | 100 | 41 | 41 |
| 1 (360) | 100 | 100 | 5 | 0 | 100 | 100 | 34.5 | 37.5 | 98 | 100 | 100 | 60 | 70 |
| 1 (394) | 100 | 100 | 0 | 6 | 100 | 100 | 12 | 24.5 | 100 | 100 | 100 | 28 | 38 |
| 1 (421) | 96 | 100 | 5 | 1.5 | 100 | 100 | 23.5 | 0 | 100 | 100 | 100 | 42 | n.a. |
| 1 (426) | 100 | 96.5 | 4 | 2 | 100 | 100 | 29 | 12 | 93 | 100 | 100 | 48 | n.a. |
| 2 (205) | 98.5 | 100 | 0 | 0 | 100 | 98 | 87 | 36.5 | 95 | 100 | 100 | 74 | 67,5 |
| 2(224) | 98 | 100 | 0 | 0 | 100 | 96 | 85 | 95 | 100 | 100 | 100 | 72 | 75 |
| 2 (228) | 100 | 100 | 13,5 | 15 | 100 | 100 | 100 | 100 | 100 | 100 | 100 | 100 | 90 |
| 2(236) | 100 | 100 | 0 | 0 | 100 | 100 | 83 | 73 | 100 | 100 | 100 | 75 | 66 |
| 2 (298) | 98.5 | 100 | 0 | 0 | 100 | 100 | 69.5 | 50 | 93.5 | 100 | 100 | 64 | 43 |
| 2 (368) | 100 | 100 | 0 | 3.5 | 100 | 100 | 64 | 47 | 91.5 | 100 | 100 | 39 | 50 |
| 3 ( 93) | 100 | 98 | 57 | 79.5 | 100 | 100 | 85 | 100 | 100 | 100 | 92.5 | 100 | n.a. |
| 3(170) | 100 | 100 | 50 | 100 | 100 | 100 | 100 | 100 | 100 | 100 | 100 | 100 | 100 |
| 3(173) | 100 | 100 | 100 | 100 | 100 | 100 | 92 | 100 | 100 | 100 | 100 | 100 | 100 |
| 3(176) | 100 | 100 | 100 | 100 | 100 | 100 | 100 | 100 | 100 | 100 | 100 | 100 | 100 |
| 3(281) | 100 | 100 | 87 | 91.5 | 100 | 100 | 100 | 100 | 100 | 100 | 100 | 100 | 96 |
| 4 ( 37) | n.a. | n.a. | n.a. | n.a. | 100 | n.a. | n.a. | 100 | n.a. | n.a. | 100 | n.a. | n.a. |
| 4 ( 69) | n.a. | 100 | n.a. | 80 | 100 | 100 | 90 | 95 | 95 | 100 | 100 | 100 | 100 |
| 4(311) | 100 | 100 | 100 | 100 | 100 | 100 | 100 | 100 | 100 | 100 | 100 | 100 | 100 |
| 4(315) | 100 | 100 | 100 | 100 | 100 | 100 | 100 | 100 | 100 | 100 | 100 | 100 | 100 |
| 4(319) | 100 | 100 | 100 | 100 | 100 | 100 | 100 | 100 | 100 | 100 | 100 | 100 | 100 |
| 4(338) | 100 | 100 | 75 | 82.5 | 100 | 100 | 100 | 94.5 | 100 | 100 | 100 | 90 | 100 |
| 4(371) | 100 | 100 | 100 | 100 | 100 | 100 | 100 | 97 | 94 | 100 | 100 | 100 | 100 |

Figure 1

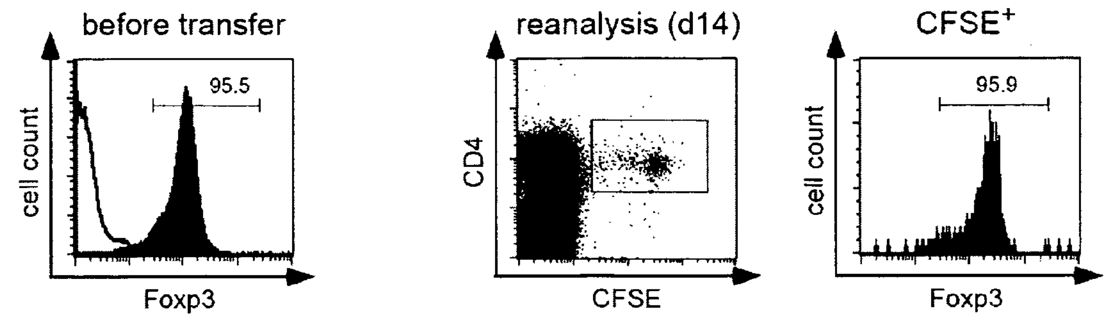


Figure 2

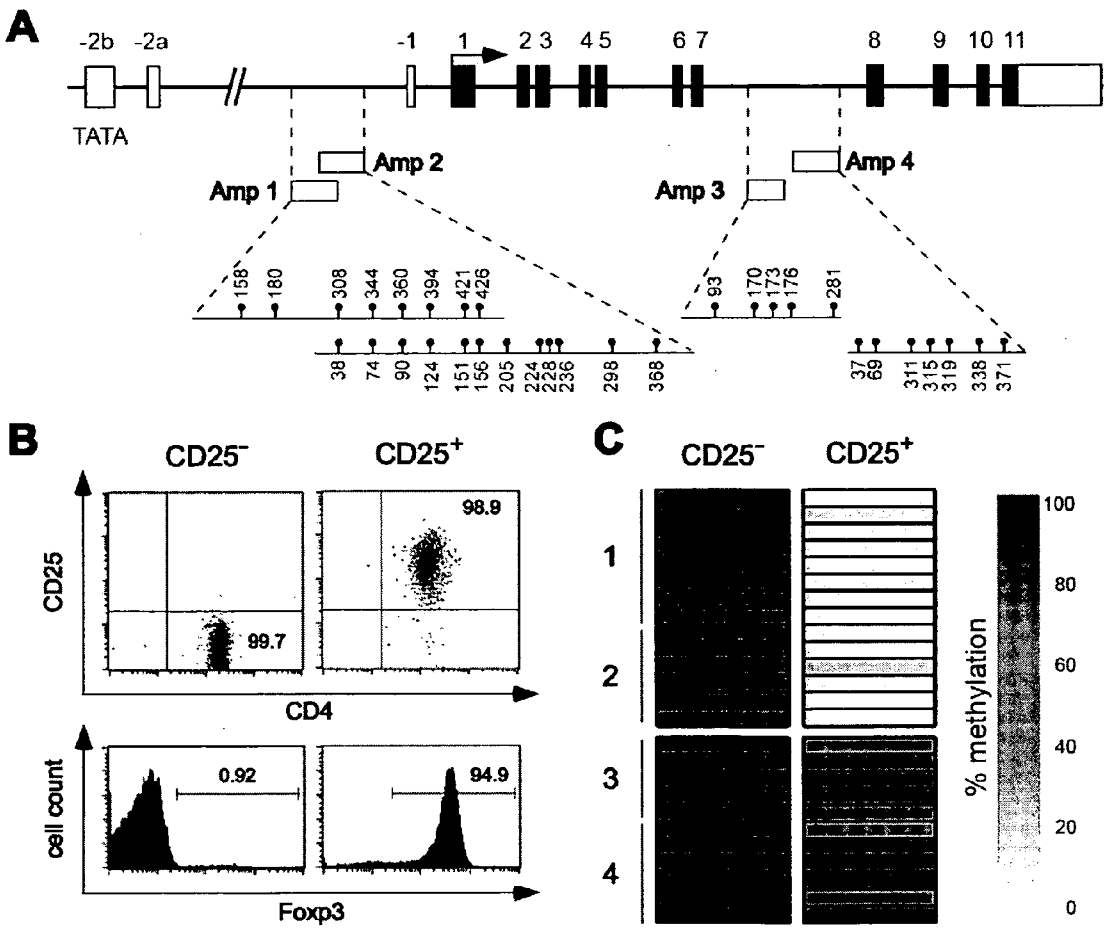

Figure 3

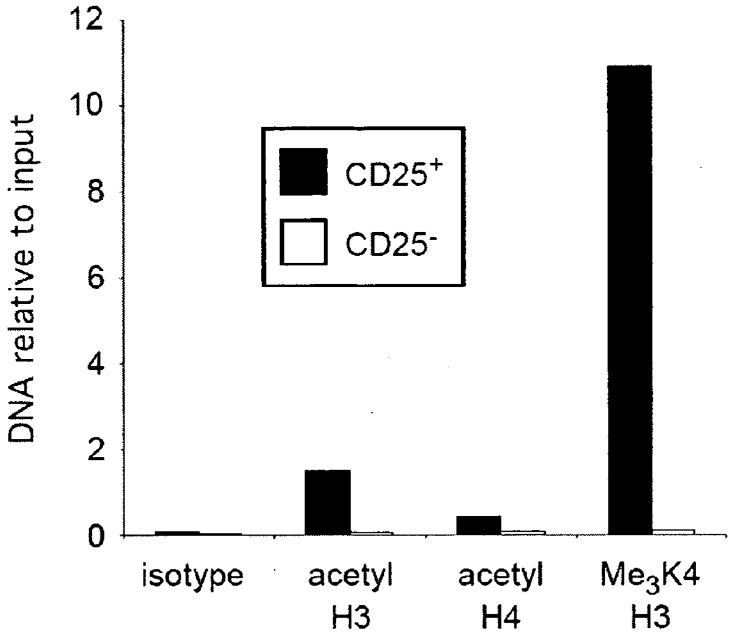


Figure 4

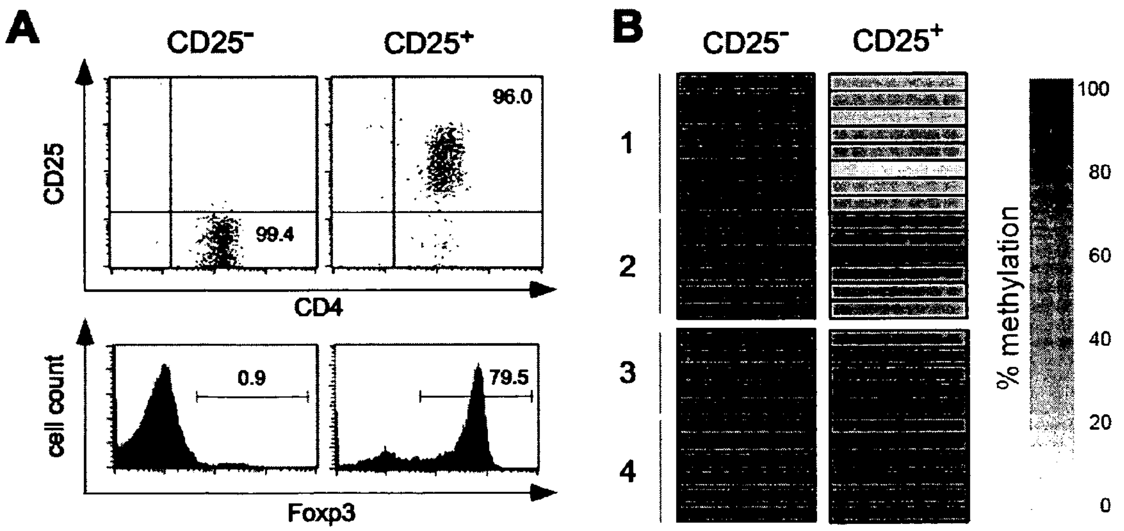

Figure 5

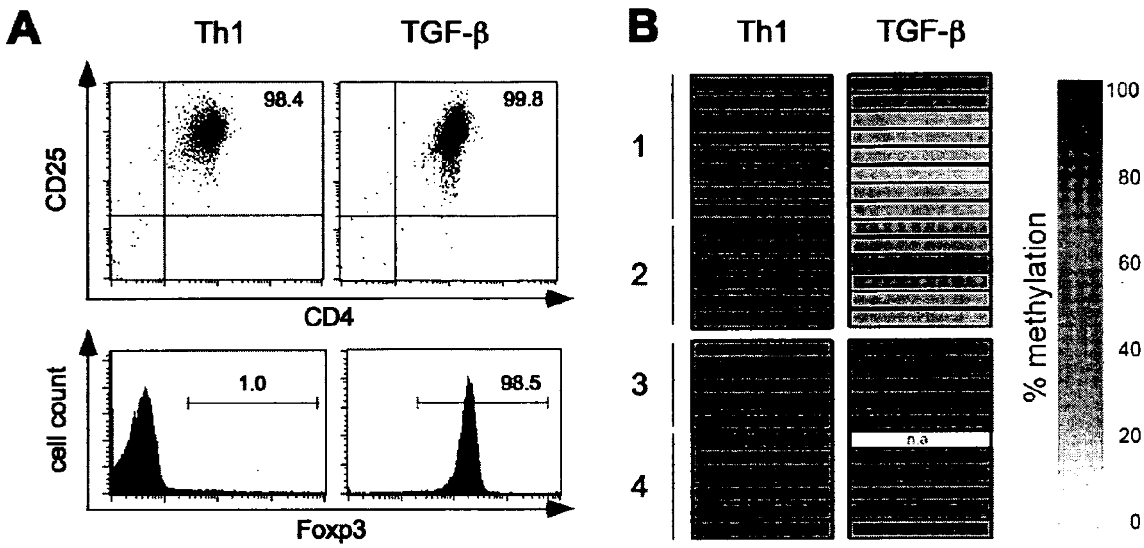
A
Th1
TGF-β
98.4
99.8
CD25
CD4
1.0
98.5
cell count
Foxp3
B
Th1
TGF-β
1
2
3
4
n.a
100
80
60
40
20
0
% methylation

Figure 6

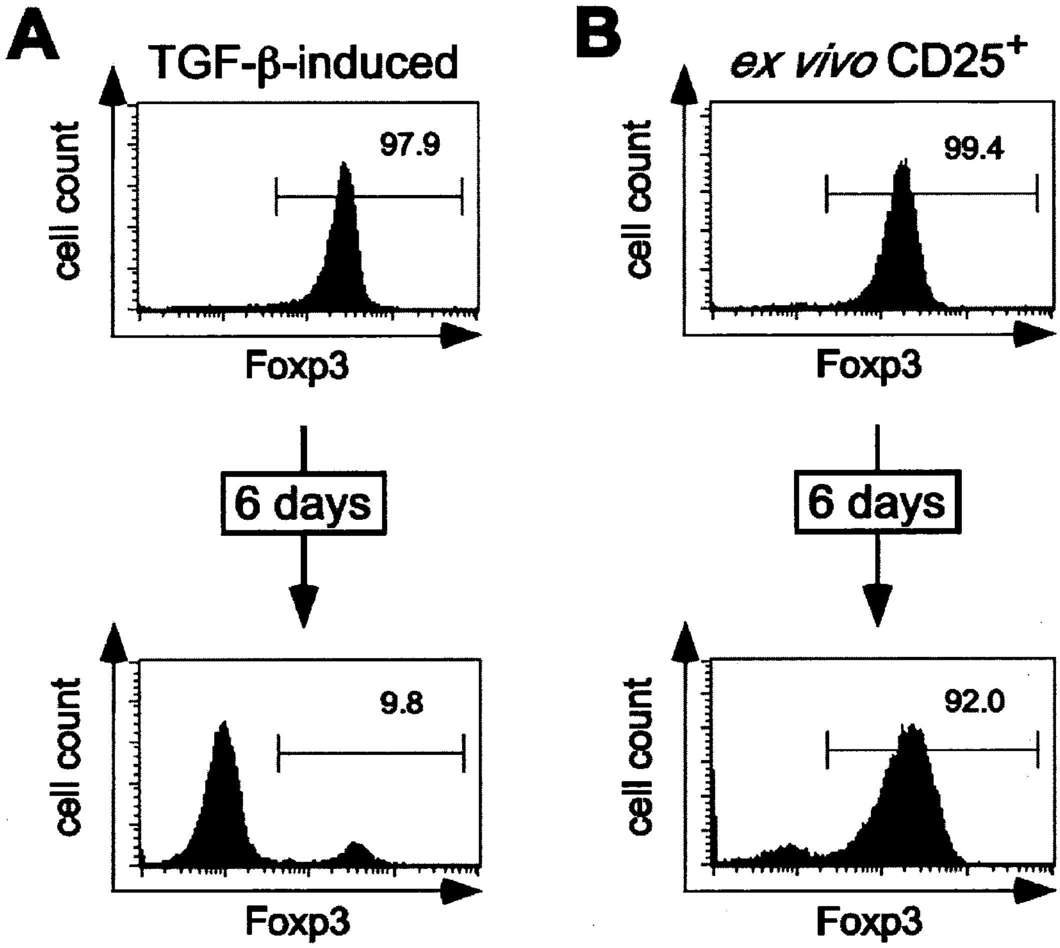
A
TGF-β-induced
cell count
97.9
Foxp3
6 days
cell count
9.8
Foxp3
B
ex vivo CD25+
cell count
99.4
Foxp3
6 days
cell count
92.0
Foxp3

Figure 7

```
  1 AGGCTGACATTCCAGAGCC-----AGCAAGAGGCCTTATGGAGTTTTAAG 45
             || .||||| ||| .|||||||||.||||.||....|||
  1 ----tg--tttccag-gcctggtaggcaagaggccctatgaagcagcaag 43

 46 CTTCCTGGCTTT-AGGTGGTTCCCATTTCTTTGGGC-TCTGGGACA---- 89
        ||.||||.|||| ||.|||||||.|....|||||.| .|.||||||
 44 ctgcctgactttcagatggttccaaggagtttggacaccagggacactgg 93

 90 TCAATACACACAGTAAGAAGGTGGATCCATGCACCCTACAGAGTCTGTGT 139
     .| ||||||.|.|.|||...|||..||.|..|||| ||.||||||||.
 94 cc--tacacatactgagactttgggacggtagaccc--cacagtctgtgg 139

140 TCTTGAGATTCTAAAATCCGTTGGCTTTGAGAAATGATATCGTACAGTTC 189
    |.||||||||||..||||.||...|.|.|||||||.|.|..||...|||
140 ttttgagattctaggatcctttaaatctaagaaatgctgttctatgattc 189

190 TGAGTTTCTGTTACTACAGCATTTGAAGACTCAAGGGGGTCTCAATATCC 239
    ||||.|.|||.|..||.|..||||||||||.|.| ||||||.||.||||.
190 tgaggtcctggtgttatactatttgaagacccca-ggggtcccagtatct 238

240 ATGAGGCCTGCCTAATACTCACCAAGCATCCAACCTTGGGCCCCTCTGGC 289
    .||..||||||||...||||.|..|||.||.||||||.||.||.|||....
239 gtggagcctgcctggcactctcagagcttcaaacctgggtcctctccaca 288

290 ATCCAAGAA-AGACAGAATCGATAGAACTTGGGTTTTG-CATGGTAGCCA 337
    |.|||||||| .|.||| .||...|||.||.|||..||| |||.||.||||
289 acccaagaagggccag-gtcttcagagctaggggcttgtcatagtggcca 337
      ATF/CREB        c-Ets-1
338 GATGGACGTCACCTACCACATCCGCTAGCACCCACATCACCCTACCTGGG 387
    |||||||.||||||||||||||||.|.|||||||||..||||||.|||||||
338 gatggacatcacctaccacatccaccagcacccatgtcaccccacctggg 387
     NF-kappaB                        STAT-1
388 CCTATCCGGCTACAGGATAGACTAGCCACTTCTCGGAACGAAACCTGTGG 437
    ||.|.||.|||.|||||.||...|||||.||||||||||||||||||||||
388 ccaagcctgctgcaggacagggcagccagttctcggaacgaaacctgtgg 437

438 GGTAGATTATCTGCCCCCTTCTCTTCCTCCTTGTTGCCGATGAAGCCCAA 487
    |||.|..|||||||||.||||||||||||||.||.||.|||||||||||..
438 ggtggggtatctgccctcttctcttcctccgtggtgtcgatgaagccgcg 487
      c-Ets-1       ATF/CREB
488 TGCATCCGGCCGCCATGACGTCAATGGCAGAAAAATCTGGCCAAGTTC-- 535
    .|||||||||||||||||||||||||||||.||||||||||||.|||| ||
488 cgcatccggccgccatgacgtcaatggcggaaaaatctgggcaag-tcgg 536
             FoxP3                       NFAT/Evi-1
536 AGGTTGTGACAACAGGGCCCAGATGTAGACCCCGATAGGAAAACATATTC 585
    .||.|||||||||||||||||||||.||||||||||||.|||||||||.||
537 gggctgtgacaacagggcccagatgcagaccccgatatgaaaacataatc 586

586 TATGTCCCAGAAACAACCTCCATACAGCTTCTAAGAAA--CAGTCAAACA 633
    |.|||||||||||||.||.||||.||||||||.||||| ||||||.|.|
587 tgtgtcccagaaacatcccccattcagcttctgagaaacccagtcagaaa 636
                               Elk-1  SMAD-4
634 GGAACGCCCCAACAGACAGTGCAGGAAGCTGGCTGGCCAGCCCAGCCCTC 683
    ||.|||.|||||||||||||||||||||||.|||||.||||||||.||||||
637 gggacgtcccaacagacagtgcaggaagccggctgcccagcccggccctc 686
                               SMAD-4
684 CAGGTCC-CTAGTACCACTAGACAGA CCATATCCAATTCAGG-- 724
    .|||||| | ||||.|.||||||||.     .  :   ..  '...
687 taggtcctc---taccccagacagatcatctccatgtccctgt 727
```

Figure 8

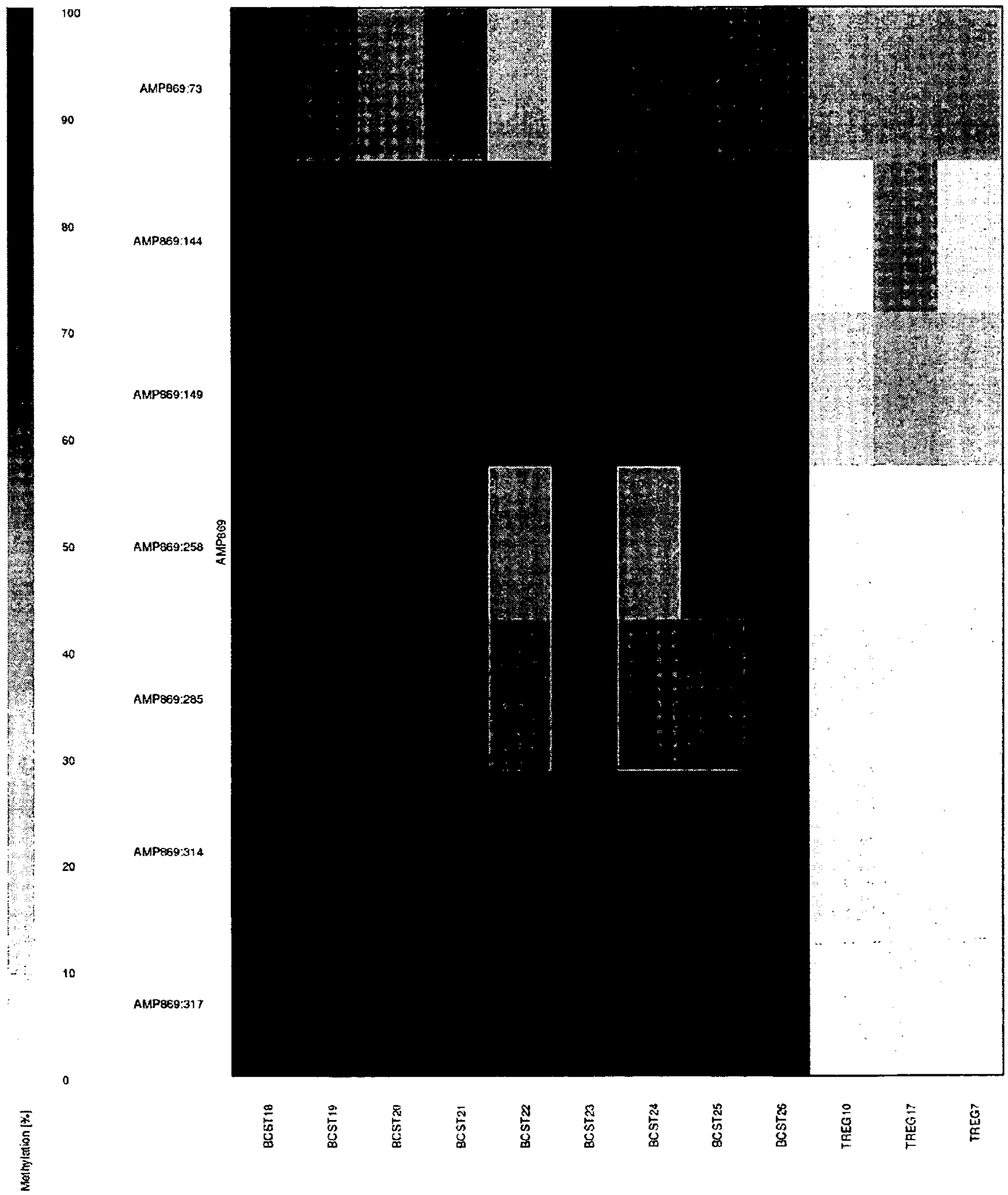
100
90
80
70
60
50
40
30
20
10
0
Methylation [%]
AMP869:73
AMP869:144
AMP869:149
AMP869:258
AMP869:285
AMP869:314
AMP869:317
AMP869
BCST18
BCST19
BCST20
BCST21
BCST22
BCST23
BCST24
BCST25
BCST26
TREG10
TREG17
TREG7

European Patent Office

# PARTIAL EUROPEAN SEARCH REPORT

which under Rule 45 of the European Patent Convention shall be considered, for the purposes of subsequent proceedings, as the European search report

Application Number
EP 07 00 4137

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A<br><br><br><br>X | WO 02/081749 A (UNIV SOUTHERN CALIFORNIA [US]; MARKL ISABEL [US]; JONES PETER A [US];) 17 October 2002 (2002-10-17)<br>* pages 10-11 *<br>* the whole document *<br>----- | 1-19<br><br><br><br>20 | INV.<br>C12Q1/68 |
| A | VIGUIER MANUELLE ET AL: "Foxp3 expressing CD4+CD25high regulatory T cells are overrepresented in human metastatic melanoma lymph nodes and inhibit the function of infiltrating T cells"<br>JOURNAL OF IMMUNOLOGY,<br>vol. 173, no. 2,<br>15 July 2004 (2004-07-15), pages 1444-1453, 1436, XP002434923<br>ISSN: 0022-1767<br>* the whole document *<br>-----<br>-/-- | 1-20 | |

TECHNICAL FIELDS SEARCHED (IPC)

C12Q

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 30 May 2007 | Dolce, Luca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
&: member of the same patent family, corresponding document

EPO FORM 1503 03.82 (P04C07)

9

European Patent Office

# PARTIAL EUROPEAN SEARCH REPORT

Application Number
EP 07 00 4137

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HORI S ET AL: "Control of regulatory T cell development by the transcription factor Foxp3" SCIENCE, AMERICAN ASSOCIATION FOR THE ADVANCEMENT OF SCIENCE,, US, vol. 299, no. 5609, 14 February 2003 (2003-02-14), pages 1057-1061, XP002316905 ISSN: 0036-8075 * the whole document * ----- | 1-20 | |
| A | KATOH M ET AL: "HUMAN FOX GENE FAMILY (REVIEW)" INTERNATIONAL JOURNAL OF ONCOLOGY, EDITORIAL ACADEMY OF THE INTERNATIONAL JOURNAL OF ONCOLOGY,, GR, vol. 25, no. 5, November 2004 (2004-11), pages 1495-1500, XP009049979 ISSN: 1019-6439 * the whole document * ----- | 1-20 | TECHNICAL FIELDS SEARCHED (IPC) |
| X,P | FLOESS STEFAN ET AL: "Epigenetic control of the foxp3 locus in regulatory T cells" PLOS BIOLOGY, vol. 5, no. 2, February 2007 (2007-02), pages 169-178, XP002434924 ISSN: 1544-9173(print) 1545-7885(ele * the whole document * ----- | 1-20 | |
| X,P | ZORN EMMANUEL ET AL: "IL-2 regulates FOXP3 expression in human CD4(+)CD25(+) regulatory T cells through a STAT-dependent mechanism and induces the expansion of these cells in vivo" BLOOD, vol. 108, no. 5, September 2006 (2006-09), pages 1571-1579, XP002434925 ISSN: 0006-4971 * the whole document * ----- | 1-20 | |

9

EPO FORM 1503 03.82 (P04C10)

European Patent Office

**INCOMPLETE SEARCH SHEET C**

Application Number
EP 07 00 4137

Although claims 16-19 are directed to a method of treatment of the human/animal body (Article 52(4) EPC), the search has been carried out and based on the alleged effects of the compound/composition.

Although claims 1 and 3 are directed to a diagnostic method practised on the human/animal body (Article 52(4) EPC), the search has been carried out and based on the alleged effects of the compound/composition.

-----

## ANNEX TO THE EUROPEAN SEARCH REPORT ON EUROPEAN PATENT APPLICATION NO. EP 07 00 4137

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2007

| Patent document cited in search report | Publication date | Patent family member(s) | Publication date |
|---|---|---|---|
| WO 02081749 A | 17-10-2002 | EP 1360317 A2 | 12-11-2003 |
| | | JP 2004527245 T | 09-09-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02090600 A

### Non-patent literature cited in the description


- **SAKAGUCHI.** *Nat Immunol,* 2005, vol. 6, 345-352
- **SAKAGUCHI, S.** Naturally arising CD4+ regulatory T cells for immunologic selftolerance and negative control of immune responses. *Annu Rev Immunol,* 2004, vol. 22, 531-562
- **FONTENOT, J.D. ; RUDENSKY, A.Y.** A well adapted regulatory contrivance: regulatory T cell development and the forkhead family transcription factor Foxp3. *Nat Immunol,* 2005, vol. 6, 331-7
- **BENNETT, C.L. et al.** The immune dysregulation, polyendocrinopathy, enteropathy, X-linked syndrome (IPEX) is caused by mutations of FOXP3. *Nat Genet,* 2001, vol. 27, 20-1
- **FONTENOT, J.D. ; GAVIN, M.A. ; RUDENSKY, A.Y.** Foxp3 programs the development and function of CD4+CD25+ regulatory T cells. *Nat Immunol,* 2003, vol. 4, 330-6
- **HORI, S. ; NOMURA, T. ; SAKAGUCHI, S.** Control of regulatory T cell development by the transcription factor Foxp3. *Science,* 2003, vol. 299, 1057-61
- **MANTEL, P.Y. et al.** Molecular Mechanisms Underlying FOXP3 Induction in Human T Cells. *J Immunol,* 2006, vol. 176, 3593-602
- **FONTENOT, J.D. et al.** Regulatory T cell lineage specification by the forkhead transcription factor foxp3. *Immunity,* 2005, vol. 22, 329-41
- **FONTENOT, J.D. ; DOOLEY, J.L. ; FARR, A.G. ; RUDENSKY, A.Y.** Developmental regulation of Foxp3 expression during ontogeny. *J Exp Med,* 2005, vol. 202, 901-6
- **THORSTENSON, K.M. ; KHORUTS, A.** Generation of anergic and potentially immunoregulatory CD25+CD4 T cells in vivo after induction of peripheral tolerance with intravenous or oral antigen. *J Immunol,* 2001, vol. 167, 188-95
- **APOSTOLOU, I. ; VON BOEHMER, H.** In vivo instruction of suppressor commitment in naive T cells. *J Exp Med,* 2004, vol. 199, 1401-8
- **KRETSCHMER, K. et al.** Inducing and expanding regulatory T cell populations by foreign antigen. *Nat Immunol,* 2005, vol. 12, 1219-27
- **CHEN, W. et al.** Conversion of peripheral CD4+CD25- naive T cells to CD4+CD25+ regulatory T cells by TGF-beta induction of transcription factor Foxp3. *J Exp Med,* 2003, vol. 198, 1875-86
- **PARK, H.B. ; PAIK, D.J. ; JANG, E. ; HONG, S. ; YOUN, J.** Acquisition of anergic and suppressive activities in transforming growth factor-beta-costimulated CD4+CD25-T cells. *Int Immunol,* 2004, vol. 16, 1203-13
- **FU, S. et al.** TGF-beta induces Foxp3 + T-regulatory cells from CD4 + CD25 -precursors. *Am J Transplant,* 2004, vol. 4, 1614-27
- **FANTINI, M.C. et al.** Cutting edge: TGF-beta induces a regulatory phenotype in CD4+CD25- T cells through Foxp3 induction and down-regulation of Smad7. *J Immunol,* 2004, vol. 172, 5149-53
- **WAN, Y.Y. ; FLAVELL, R.A.** Identifying Foxp3-expressing suppressor T cells with a bicistronic reporter. *Proc Natl Acad Sci U S A,* 2005, vol. 102, 5126-31
- **ANSEL, K.M. ; LEE, D.U. ; RAO, A.** An epigenetic view of helper T cell differentiation. *Nat Immunol,* 2003, vol. 4, 616-23
- **REINER, S.L.** Epigenetic control in the immune response. *Hum Mol Genet,* 2005, vol. 14 (1), R41-6
- **TYKOCINSKI, L.O. et al.** A critical control element for interleukin-4 memory expression in T helper lymphocytes. *J Biol Chem,* 2005, vol. 280, 28177-85
- **CHEN L ; COHEN AC ; LEWIS DB.** Impaired Allogeneic Activation and T-helper 1 Differentiation of Human Cord Blood Naive CD4 T Cells. *Biol Blood Marrow Transplant.,* February 2006, vol. 12 (2), 160-71
- **BOUCHE, N. ; SCHARLAT, A. ; SNEDDEN, W. ; BOUCHEZ, D. ; FROMM, H.** A novel family of calmodulin-binding transcription activators in multicellular organisms. *J. Biol. Chem.,* 2002, vol. 277 (24), 21851-21861

- **K. O. HENRICH ; M. FISCHER ; D. MERTENS ; A. BENNER ; R. WIEDEMEYER ; B. BRORS ; A. OBERTHUER ; F. BERTHOLD ; J. S. WEI ; J. KHAN.** Reduced expression of CAMTA1 correlates with adverse outcome in neuroblastoma patients. *Clin Cancer Res,* January 2006, vol. 12 (1), 131-138
- **ANTEQUERA ; BIRD.** *Proc Natl Acad Sci U S A.,* 1993, vol. 90, 11995-9
- **JONES ; LAIRD.** *Nature Genetics,* 1999, vol. 21, 163-167
- **ESTELLER.** *Oncogene,* 2002, vol. 21, 5427-5440
- **LAIRD.** *Nature Reviews/Cancer,* 2003, vol. 3, 253-266
- **ERLICH.** *J Cellular Chem,* 2003, vol. 88, 899-910
- **BIRD.** *Genes and Dev,* 2002, vol. 16, 6-21
- **LI.** *Nature Reviews/Genetics,* 2002, vol. 3, 662-673
- **ANSEL et al.** *Nature Immunol,* 2003, vol. 4, 616-623
- **ATTWOOD et al.** *CMLS,* 2002, vol. 59, 241-257
- **LEE et al.** *Immunity,* 2002, vol. 16, 649-660
- **BRUNIQUEL ; SCHWARTZ.** *Nat Immunol.,* 2003, vol. 4, 235-40
- **DIETL J ; ENGEL JB ; WISCHHUSEN J.** The role of regulatory T cells in ovarian cancer. *Int J Gynecol Cancer,* 14 February 2007
- **CURIEL TJ ; COUKOS G ; ZOU L ; ALVAREZ X ; CHENG P ; MOTTRAM P ; EVDEMON-HOGAN M ; CONEJO-GARCIA JR ; ZHANG L ; BUROW M.** Specific recruitment of regulatory T cells in ovarian carcinoma fosters immune privilege and predicts reduced survival. *Nat Med.,* 22 August 2004, vol. 10 (9), 942-9
- **KOBAYASHI N ; HIRAOKA N ; YAMAGAMI W ; OJIMA H ; KANAI Y ; KOSUGE T ; NAKAJIMA A ; HIROHASHI S.** FOXP3+ regulatory T cells affect the development and progression of hepatocarcinogenesis. *Clin Cancer Res.,* 01 February 2007, vol. 13 (3), 902-11
- **BRUNKOW ME ; JEFFERY EW ; HJERRILD KA ; PAEPER B ; CLARK LB ; YASAYKO SA ; WILKINSON JE ; GALAS D ; ZIEGLER SF ; RAMSDELL F.** Disruption of a new forkhead/winged-helix protein, scurfin, results in the fatal lymphoproliferative disorder of the scurfy mouse. *Nat Genet.,* January 2001, vol. 27 (1), 68-73
- **FONTENOT, J.D. ; RUDENSKY, A.Y.** A well adapted regulatory contrivance: regulatory T cell development and the forkhead family transcription factor Foxp3. *Nat Immunol,* 2005, vol. 6, 331-7
- **BASSUNY, W.M. et al.** A functional polymorphism in the promoter/enhancer region of the FOXP3/Scurfin gene associated with type I diabetes. *Immunogenetics,* 2003, vol. 55, 149-56
- **BARATELLI, F. et al.** Prostaglandin E2 induces FOXP3 gene expression and T regulatory cell function in human CD4+ T cells. *J Immunol,* 2005, vol. 175, 1483-90
- **BRUNIQUEL, D. ; SCHWARTZ, R.H.** Selective, stable demethylation of the interleukin-2 gene enhances transcription by an active process. *Nat Immunol,* 2003, vol. 4, 235-40
- **SCHAEFER, C. et al.** Characteristics of CD4+CD25+ regulatory T cells in the peripheral circulation of patients with head and neck cancer. *Br J Cancer,* 2005, vol. 92, 913-20
- **ORMANDY, L.A. et al.** Increased populations of regulatory T cells in peripheral blood of patients with hepatocellular carcinoma. *Cancer Res,* 2005, vol. 65, 2457-64
- **RONCADOR, G. et al.** FOXP3, a selective marker for a subset of adult T-cell leukaemia/lymphoma. *Leukemia,* 2005, vol. 19, 2247-53
- **CURIEL, T.J. et al.** Specific recruitment of regulatory T cells in ovarian carcinoma fosters immune privilege and predicts reduced survival. *Nat Med,* 2004, vol. 10, 942-9
- **WOLF, D. et al.** The expression of the regulatory T cell-specific forkhead box transcription factor FoxP3 is associated with poor prognosis in ovarian cancer. *Clin Cancer Res,* 2005, vol. 11, 8326-31
- **BARON, U. et al.** DNA Methylation Analysis as a Tool for Cell Typing. *Epigenetics,* 2006, vol. 1, 55-60
- **HUEHN, J. et al.** Developmental stage, phenotype and migration distinguish naive- and effector/memory-like CD4+ regulatory T cells. *J Exp Med,* 2004, vol. 199, 303-313
- **SIEGMUND, K. et al.** Migration matters: regulatory T cell compartmentalization determines suppressive activity in vivo. *Blood,* 2005, vol. 106, 3097-3104
- **OLEK, A. ; OSWALD, J. ; WALTER, J.** A modified and improved method for bisulphite based cytosine methylation analysis. *Nucleic Acids Res,* 1996, vol. 24, 5064-6
- **LEWIN, J. ; SCHMITT, A.O. ; ADORJAN, P. ; HILDMANN, T. ; PIEPENBROCK, C.** Quantitative DNA methylation analysis based on four-dye trace data from direct sequencing of PCR amplificates. *Bioinformatics,* 2004, vol. 20, 3005-12
- **MATYS, V. et al.** TRANSFAC and its module TRANSCompel: transcriptional gene regulation in eukaryotes. *Nucleic Acids Res,* 2006, vol. 34, D 108-10
- **KEL, A.E. et al.** MATCH: A tool for searching transcription factor binding sites in DNA sequences. *Nucleic Acids Res,* 2003, vol. 31, 3576-9

- **SUFFIA, I.J. ; RECKLING, S.K. ; PICCIRILLO, C.A. ; GOLDSZMID, R.S. ; BELKAID, Y.** Infected site-restricted Foxp3+ natural regulatory T cells are specific for microbial antigens. *J Exp Med,* 2006, vol. 203, 777-88
- **BRUNKOW, M.E. et al.** Disruption of a new forkhead/winged-helix protein, scurfin, results in the fatal lymphoproliferative disorder of the scurfy mouse. *Nat Genet,* 2001, vol. 27, 68-73
- **BRUNKOW, M.E. et al.** Disruption of a new forkhead/winged-helix protein, scurfin, results in the fatal lymphoproliferative disorder of the scurfy mouse. *Nat Genet,* 2001, vol. 27, 68-73
- **DOBOSY, J.R. ; SELKER, E.U.** Emerging connections between DNA methylation and histone acetylation. *Cell Mol Life Sci,* 2001, vol. 58, 721-7
- **FU, S. et al.** TGF-beta induces Foxp3 + T-regulatory cells from CD4 + CD25 - precursors. *Am J Transplant,* 2004, vol. 4, 1614-27
- **FANTINI, M.C. et al.** Cutting edge: TGF-beta induces a regulatory phenotype in CD4+CD25- T cells through Foxp3 induction and down-regulation of Smad7. *J Immunol,* 2004, vol. 172, 5149-53
- **FANTINI, M.C. et al.** TGF-{beta} induced Foxp3+ regulatory T cells suppress Th1-mediated experimental colitis. *Gut,* 2005

SEQUENCE LISTING

<110> Epiontis GmbH

<120> Epigenetic modification of the loci for camta1 and/or foxp3 as a marker for cancer treatment

<130> FB17768

<160> 11

<170> PatentIn version 3.4

<210> 1
<211> 21
<212> DNA
<213> Mus musculus

<400> 1
aggaagagaa gggggtagat a 21

<210> 2
<211> 22
<212> DNA
<213> Mus musculus

<400> 2
aaactaacat tccaaaacca ac 22

<210> 3
<211> 22
<212> DNA
<213> Mus musculus

<400> 3
atttgaattg gatatggttt gt 22

<210> 4
<211> 22
<212> DNA
<213> Mus musculus

<400> 4
aaccttaaac ccctctaaca tc 22

<210> 5
<211> 22
<212> DNA
<213> Mus musculus

<400> 5
agaggttgaa ggaggagtat tt 22

<210> 6
<211> 22
<212> DNA
<213> Mus musculus

<400> 6
actatctatc caattcccca ac 22

<210> 7
<211> 22
<212> DNA
<213> Mus musculus

<400> 7
tggttgtttt ggagtttagt gt 22

<210> 8
<211> 22
<212> DNA
<213> Mus musculus

<400> 8
cacttttcta ccttccacaa at 22

<210> 9
<211> 17
<212> DNA
<213> Mus musculus

<400> 9
gactcaaggg ggtctca 17

<210> 10
<211> 17
<212> DNA
<213> Mus musculus

<400> 10
ttgggcttca tcggcaa 17

<210> 11
<211> 8457
<212> DNA
<213> Homo sapiens

<400> 11
gacgctcctc ccggagagta gtgagacccc tggtgcgggg cgattggcgg cgggagcgat 60

gagtggcagc cgcacggccc aacgggagct gtgcgtgggc cgcggggcgg ggccagggcg 120

ggtgcgcggc ggcggcgggg tggctgggcc ggcggcggcg gcggtacgag gcgcgcgctc 180

ggggtcccgg tcgcgaggag gaggaggatg tggcgcgcgg aggggaaatg gctgccgaaa 240

acaagccgga agagcgtttc ccaaagtgta ttctgcggaa ctagcaccta ctgtgttctc 300
aacaccgtgc cacctataga agatgatcat gggaacagca atagtagtca tgtaaaaatc 360
tttttaccga aaaagctgct tgaatgtctg ccgaaatgtt caagtttacc aaaagagagg 420
caccgctgga acactaatga ggaaattgca gcttatttaa taacatttga gaaacacgaa 480
gaatggctaa ccacctcccc taagacaaga ccacagaatg gctcaatgat actctacaac 540
aggaagaaag tgaaatacag gaaagatggg tattgctgga aaaagaggaa agatgggaaa 600
acgaccagag aggaccacat gaaactcaag gtccagggag tggagtgctt gtacggctgc 660
tatgtccatt cctccatcat ccccaccttc caccggaggt gctactggct ccttcagaac 720
cccgacatcg tcctggtgca ctacctgaac gtgccggcca tcgaggactg cggcaagcct 780
tgcggcccca tcctctgctc catcaacacc gacaagaagg agtgggcgaa atggacgaaa 840
gaagagctca tcgggcagct gaaacccatg ttccatggca tcaagtggac ctgcagcaat 900
gggaacagca gctcaggctt ctcggtggaa cagctggtgc agcagatcct cgacagccac 960
cagaccaagc cccagccgcg gacccacaac tgcctctgca ccggcagcct gggagctggc 1020
ggcagcgtgc atcacaagtg taacagcgcc aaacaccgca tcatctcgcc caaggtggag 1080
ccacggacag gggggtacgg gagccactcg gaggtgcagc acaatgacgt gtcggagggc 1140
aagcacgagc acagccacag caagggctcc agccgtgaga agaggaacgg caaggtggcc 1200
aagcccgtgc tcctgcacca gagcagcacc gaggtctcct ccaccaacca ggtggaagtc 1260
cccgacacca cccagagctc ccctgtgtcc atcagcagcg ggctcaacag cgacccggac 1320
atggtggaca gcccggtggt cacaggtgtg tccggtatgg cggtggcctc tgtgatgggg 1380
agcttgtccc agagcgccac ggtgttcatg tcagaggtca ccaatgaggc cgtgtacacc 1440
atgtccccca ccgctggccc caaccaccac ctcctctcac ctgacgcctc tcagggcctc 1500
gtcctggccg tgagctctga tggccacaag ttcgcctttc ccaccacggg cagctcagag 1560
agcctgtcca tgctgcccac caacgtgtcc gaagagctgg tcctctccac caccctcgac 1620
ggtggccgga agattccaga aaccaccatg aactttgacc ccgactgttt ccttaataac 1680
ccaaagcagg gccagacgta cgggggtgga ggcctgaaag ccgagatggt cagctccaac 1740
atccggcact cgccacccgg ggagcggagc ttcagcttta ccaccgtcct caccaaggag 1800
atcaagaccg aggacacctc cttcgagcag cagatggcca aagaagcgta ctcctcctcc 1860
gcggcggctg tggcagccag ctccctcacc ctgaccgccg gctccagcct cctgccgtcg 1920
ggcggcggcc tgagtcccag caccaccctg gagcagatgg acttcagcgc catcgactcc 1980

```
aacaaggact acacgtccag cttcagccag acgggccaca gcccccacat ccaccagacc 2040
ccctccccga gcttcttcct gcaggacgcc agcaaacccc tccccgtcga gcagaacacc 2100
cacagcagcc tgagtgactc tgggggcacc ttcgtgatgc ccacggtgaa aacggaggcc 2160
tcgtcccaaa ccagctcctg cagcggtcac gtggagacgc ggatcgagtc cacttcctcc 2220
ctccacctca tgcagttcca ggccaacttc caggccatga cggcagaagg ggaggtcacc 2280
atggagacct cgcaggcggc ggaagggagc gaggtcctgc tcaagtctgg ggagctgcag 2340
gcttgcagct ctgagcacta cctgcagccg gagaccaacg gggtaatccg aagcgccggc 2400
ggcgtcccca tcctcccggg caacgtggtg cagggactct accccgtggc ccagcccagc 2460
ctcggcaacg cctccaacat ggagctcagc ctggaccact ttgacatctc cttcagcaac 2520
cagttctccg acctgatcaa cgacttcatc tccgtggagg ggggcagcag caccatctat 2580
gggcaccagc tggtgtcggg ggacagcacg gcgctctcac agtcagagga cggggcgcgg 2640
gccccttca cccaggcaga gatgtgcctc ccctgctgta gcccccagca gggtagcctg 2700
cagctgagca gctcggaggg cggggccagc accatggcct acatgcacgt cgccgaggtg 2760
gtctcggccg cctcggccca gggcaccta ggcatgctgc agcagagcgg acgggtgttc 2820
atggtgaccg actactcccc agagtggtct tacccagagg gaggagtgaa ggtcctcatc 2880
acaggcccgt ggcaagaagc cagcaataac tacagctgcc tgtttgacca gatctcagtg 2940
cctgcatccc tgattcagcc tggggtgctg cgctgctact gcccagccca tgacactggt 3000
cttgtgaccc tacaagttgc cttcaacaac cagatcatct ccaactcggt ggtgtttgag 3060
tacaaagccc gggctctgcc cacgctccct tcctcccagc acgactggct gtcgttggac 3120
gataaccagt tcaggatgtc catcctggaa cgactggagc agatggagag gaggatggcc 3180
gagatgacgg ggtcccagca gcacaaacag gcgagcggag gcggcagcag tggaggcggc 3240
agcgggagcg ggaatggagg gagccaggca cagtgtgctt ctgggactgg ggccttgggg 3300
agctgctttg agagccgtgt ggtcgtggta tgcgagaaga tgatgagccg agcctgctgg 3360
gcgaagtcca agcacttgat ccactcaaag actttccgcg gaatgaccct actccacctg 3420
gccgctgccc agggctatgc caccctaatc cagaccctca tcaaatggcg tacaaagcac 3480
gcggatagca ttgacctgga actggaagtt gacccccttga atgtggacca cttctcctgt 3540
actcctctga tgtgggcgtg tgccctaggg cacttggaag ctgccgtcgt gctgtacaag 3600
tgggaccgtc gggccatctc gattcccgac tctctaggaa ggctgccttt gggaattgcc 3660
aggtcacggg gtcatgtgaa attagcagag tgtctggagc acctgcagag agatgagcag 3720
```

gctcagctgg gacagaaccc cagaatccac tgtcctgcaa gcgaagagcc cagcacagag 3780
agctggatgg cccagtggca cagcgaagcc atcagctctc cagaaatacc caagggagtc 3840
actgttattg caagcaccaa cccagagctg agaagacctc gttctgaacc ctctaattac 3900
tacagcagtg agagccacaa agattatccg gctcccaaaa agcataaatt gaaccctgag 3960
tacttccaga caaggcagga gaagctgctt cccactgcac tgagtctgga agagccaaat 4020
atcaggaagc aaagccctag ttctaagcag tctgtccccg agacactcag ccccagtgaa 4080
ggagtgaggg acttcagccg ggaactctcc cctcccactc cagagactgc agcatttcaa 4140
gcctctggat ctcagcctgt aggaaagtgg aattccaaag atctttacat tggtgtgtct 4200
acagtacagg tgactggaaa tccgaagggg accagtgtag gaaaggaggc agcaccttca 4260
caggtgcgtc cacgggaacc aatgagtgtc ctgatgatgg ctaacagaga ggtggtgaat 4320
acagagctgg ggtcctaccg tgatagtgca gaaaatgaag aatgcggcca gcccatggat 4380
gacatacagg tgaacatgat gaccttggca gaacacatta ttgaagccac acctgaccga 4440
atcaagcagg agaattttgt gcccatggag tcctcaggat tggaaagaac agaccctgcc 4500
accattagca gtacaatgag ctggctggcc agttatctag cggatgctga ctgccttccc 4560
agtgctgccc agatccgaag tgcatataac gagcctctaa ccccttcttc taataccagc 4620
ttgagccctg ttggctctcc cgtcagtgaa atcgctttcg agaaacctaa ccttccctcc 4680
gccgcggatt ggtcagaatt cctgagtgca tctaccagtg agaaggtaga gaatgagttt 4740
gctcagctca ctctgtctga tcatgaacag agagaactct atgaggctgc caggcttgtc 4800
cagacagctt tccggaaata caagggccga cccttgcggg aacagcaaga agtagctgct 4860
gctgttattc agcgttgtta cagaaaatat aaacagtacg cactttataa aaagatgaca 4920
caggctgcca tccttatcca gagcaaattc cgaagttact atgaacaaaa aaaattccag 4980
cagagccgac gggctgctgt gctcatccaa aagtactacc gaagttataa gaaatgtggc 5040
aaaagacggc aggctcgccg gacggctgtg attgtacaac agaaactcag gagcagtttg 5100
ctaaccaaaa agcaggatca agctgctcga aaaataatga ggtttcttcg ccgctgtcgc 5160
cacagccccc tggtggacca taggctgtac aaaaggagtg aaagaattga aaaaggccaa 5220
ggaacttgaa gacatacagc agcatccctt agcaatgtga cattgctttt cagactgttt 5280
tcatttctgt ttttagcaga gacatgcaac aacaacacac acgcacacac gcacacacac 5340
acacgtacac acacatacaa aatccctctg cagttttggg gagatcagct gcaggatttt 5400
aacaggaatg ttttggtcat tgcatttgca ctttcatgga caacttttaa tttgatcagc 5460

aagacatctt ggaactcaat cttctgttgg atcacgggaa atcaagacac ccaggaggaa 5520
ttgaaagagg cttcctcttc tcaggaagaa gccatttcct tctcatatag ggctgtattc 5580
aaacatcgtg tggaactgta caaatattta taccaaaaat atagataaga aaaggtgggg 5640
ctatactagc aacaaaaaaa gaatgctgtt cctgcacctg ccggttattt ccaagaagct 5700
gaatctttgg gactgattct cagtggaggg cttagatcat acaaaaatct ttattgggtc 5760
cgtgtgttct catttccttc actgtttatt tttgtttgtt tgtttgtttg ttttaatctc 5820
tacagcacat ttaatgcaac ttttgaaatc tgcaggtttt taatgtcttg tggaaatttg 5880
cagaggggca ggtgtgtggt aaacgggtaa tgcatgggaa ataatgagaa gcagctcaca 5940
gagtttaaac tattttcttg tccccaccac cttccaagaa cctgcgaggg tagtaatcat 6000
cttgtcccct ttttcatgtt cagcacttta atttttttgc cttactttca tgtgcaatga 6060
gaattactta agaattggta acgcatgtag ccttttttag taaccttgga agctgtagta 6120
attctaagga atcatgaacc ttgcctggac atttgccacc taaacgatca gtgtggtgct 6180
gcgttctggc cagtaaattc catgtttttg gctatatctc atccaaactg agcagtttct 6240
gtgtatatat agaaggtaga aatgaaaagt gagaaaatat ttgaaaggga ttatattaat 6300
tgctaaatat tttattcaca aaggtcaata acatggcaag ataaaattat ttgtatagtt 6360
ttgtctgaat gagcgagaaa aatgtggatg tactgtttgt atatattgta tatattaaaa 6420
cagagatatg tgcatgaaat caagaaaaaa gaaatgaaca aaagcaaagc attagtggct 6480
atggtctgta aaatgaaaca aaaaaacttt atttcactat aagagtactt tattttaaat 6540
gttctttagg agaacatttt gctaaagcat gactaaactg caaaaaaaaa aaaagagcta 6600
ctgtatttag acttaggaaa aaaggcagag taacattact taaaaaaaaa aggatatgtt 6660
tacatttaat tttggctacc aggagtttag tttattttat ttaaaatttt tttgccaatg 6720
gtgccaagta atgtgaatgc taatactgct taagaaaatt aagttacttt tgcaaaacag 6780
ataatcataa gatgaatcag tatgtagctt aacaccatcc actcactcca acaaagaaca 6840
cttagaatga taaaaaaaaa aaaaaaaaac ctgacaaaag aaatagtatg aaaagtagaa 6900
aaatgtcacg tttccatatc tcctgctgga aatcagaaaa tataataaaa ttgcacaaaa 6960
aaaaaaatga aaaagatgca gactggtctt ttagagacgg catggtatat tactatttcc 7020
acataatgag gagccaaaga aatctgatgt ttttaacaat taaactgcta atgttaaatt 7080
gagagaataa agttcgtatt tgctgatgcc agtttaaaat tcccaggtta cgtctgagga 7140
tcagttggtg taaagctgag atgtttttc ttggttctgg ctgccaactg tgagttaaaa 7200

```
ctcaaggctt gttgtgaagc ctaaaaatat tcacaaataa gcttttaaac tggtgtcttt   7260
ggaaggaagg tagatacaaa aagattgtgg taaaaactgg ggtcagtgct cttggtgcct   7320
tttctataat tgtacttgtt ttttaattac ttcctttcac tgccaacctc gaattactgt   7380
acagtatatg tctttctgct tgtgatcagc tttgacaaca gtgacagccc cacaactagt   7440
agccacctgt acatttgtaa actgacctga ctccattttg tttttaaatg tgtgggttat   7500
gttgcagctg ttgcagtccc ccagatacct attattttac acaatttgac ctataggagg   7560
acactgagta atttacaaac acaactgcat tcataaatgg gaatagaacg tgaaagccag   7620
ctctttcaga atatcctcta ttaatactga atttagatat ctttattcca tttattatgg   7680
tacaaataac tgatgtttta accagagtaa tgacctcagt ggatttgctt taaccctcac   7740
attttttttt taatgtttca catgttacat tattagctga atacgttaga aaatgacaga   7800
tggtagagac ttccatagaa ttaagagggt tctcatggag gggataggaa gtaggtttaa   7860
agcctaccag tgtaacctac cagtacaact gtgaatccta ggcgaggcaa aaatgcactt   7920
ccactgaaac gaagcatttc tgaccgcttt ttcttggtta tgaatcttaa tttcgaatat   7980
aagatgatag gtaagcgcag ctttcttgga taatctgaat tcccaagtgc caggagtagg   8040
atttcattat aaaattaata gctaatctta ttctatctcc tgaagattta attgctattg   8100
ttacccattc gaaatcagct gtactgtgtg aacgaaataa agacaataat acgaacccct   8160
ctctggctgc acggtcgctt atggcagttc cacacagtag ttggcgccca atggggggtc   8220
cctgagactt gcatgtaaaa ctagtctaga tgtcttcttt ttttgtaagt tttatttttg   8280
taattgtgca tgtaaagcat cattgaatca atggatctgt tgaagaactc agctgctgga   8340
aaccatgcaa aatgttttga attgccctta aaatatggaa aatgttttct gaatgcttta   8400
tattctttct gctgtaaatt aaaaatgaag aaaatttccc caaaaaaaaa aaaaaaa      8457
```